(19) Europäisches Patentamt
European Patent Office
Office européen des brevets

(11) EP 2 459 166 B1

(12) # FASCICULE DE BREVET EUROPEEN

(45) Date de publication et mention de la délivrance du brevet:
**12.10.2016 Bulletin 2016/41**

(21) Numéro de dépôt: **10740198.6**

(22) Date de dépôt: **27.07.2010**

(51) Int Cl.:
***A61Q 19/00*** (2006.01) ***A61K 8/97*** (2006.01)

(86) Numéro de dépôt international:
**PCT/EP2010/060873**

(87) Numéro de publication internationale:
**WO 2011/012612 (03.02.2011 Gazette 2011/05)**

(54) **EXTRAIT DU FRUIT DE SCHIZANDRA SPHENANTHERA ET COMPOSITIONS COSMETIQUES, DERMATOLOGIQUES ET NUTRACEUTIQUES LE COMPRENANT**

SCHISANDRA-SPHENANTHERA-FRUCHTEXTRAKT UND KOSMETISCHE, DERMATOLOGISCHE SOWIE NUTRAZEUTISCHE ZUSAMMENSETZUNGEN DAMIT

SCHISANDRA SPHENANTHERA FRUIT EXTRACT AND COSMETIC, DERMATOLOGICAL AND NUTRACEUTICAL COMPOSITIONS COMPRISING SAME

(84) Etats contractants désignés:
**AL AT BE BG CH CY CZ DE DK EE ES FI FR GB GR HR HU IE IS IT LI LT LU LV MC MK MT NL NO PL PT RO SE SI SK SM TR**

(30) Priorité: **30.07.2009 FR 0955344**

(43) Date de publication de la demande:
**06.06.2012 Bulletin 2012/23**

(73) Titulaire: **Laboratoires Expanscience**
**92400 Courbevoie (FR)**

(72) Inventeurs:
- **GARNIER, Sébastien**
  **F-28130 Pierres (FR)**
- **MSIKA, Philippe**
  **F-78000 Versailles (FR)**

(74) Mandataire: **Regimbeau**
**139, rue Vendôme**
**69477 Lyon Cedex 06 (FR)**

(56) Documents cités:
**WO-A2-2005/044289 FR-A1- 2 910 815**

- **"Schisandrafrüchte CO2-to Extrakt Varietät Sphenanthera", FLAVEX Naturextrakte GmbH , 15 mars 2006 (2006-03-15), XP002576905, Extrait de l'Internet: URL:http://www.flavex.com/fileadmin/flavex .de/user_upload/Spezifikation/Deutsch/Spezi_Schisandrafruechte_CO2-to_Extrakt_164_001.pdf [extrait le 2010-04-07]**
- **DR. K. W. QUIRIN: "Supercritical Schisandra Extracts - a New Concept for Personal Care Cosmetics", COSMETIC SCIENCE TECHNOLOGY, 2008, XP002576906,**
- **HUYKE C ET AL.: "Composition and Biological Activity of Different extracts from Schisandra sphenanthera and Schisandra chinensis", PLANTA MED, vol. 73, 5 juillet 2007 (2007-07-05), pages 1116-1126, XP002576907, cité dans la demande**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 2 459 166 B1

## Description

### INTRODUCTION

L'invention se rapporte à une composition cosmétique, dermatologique ou nutraceutique comprenant un excipient approprié et un extrait de *Schizandra sphenanthera.*

### La plante *Schizandra sphenanthera*

De nom botanique *Schizandra sphenanthera*, cette plante appartient à la classe des *Magnoliopsida* et à l'ordre des *Magnoliales.* La famille botanique est celle des *Schisandraceae*. Le nom latin complet est *Schisandra sphenanthera* Rehd. et Wils. Les noms usuels de la plante sont : schisandre à fleurs orangées (en France), southern schisandra, lemon wood (en Angleterre), hua zhong wu wei zi, nan wu wie zi (en Chine).

Ces arbustes sont originaires du Nord de la Chine et des régions adjacentes de Russie et Corée. Il existe dans le monde à peu près 25 espèces appartenant au genre *Schisandra*. Environ 16 d'entre elles sont chinoises. Deux sont officiellement reconnues comme médicinales en Chine, *S. chinensis* et *S. sphenanthera* [He et al., 1997]. Leurs baies sont utilisées en médecine traditionnelle chinoise pour le traitement de la toux, de l'asthme, des sueurs nocturnes, des éjaculations nocturnes et de la diarrhée chronique. Elles sont aussi utilisées comme tonifiants et pour le traitement de la fatigue chronique.

Le terme *Schizandra* utilisé tel quel peut désigner deux plantes différentes que sont *Schizandra chinensis* et *Schizandra sphenanthera.* Longtemps ces deux espèces ont été considérées comme équivalentes, elles pouvaient être désignées selon leur origine « Northern *Schizandra »* pour *Schizandra chinensis* et « Southern *Schizandra* » pour *Schizandra sphenanthera.*

*Schizandra sphenanthera* est une plante dioïque (pieds aux fleurs mâles et femelles distincts). Le fruit se présente sous forme de grappe pendante qui rappelle un peu celle du groseillier. Elle est pourvue d'un pédoncule nu dans sa partie supérieure (environ 5-10 cm) qui est recouvert dans sa partie inférieure par des baies rouges vifs, un peu plus grosses, plus compactes et plus fermes que la groseille. La graine, sphérique, mesure quelques millimètres.

### Caractéristiques du fruit

Si le fruit de *S. chinensis* a été l'objet d'une littérature conséquente (utilisation traditionnelle, compositions chimiques, effets pharmacologiques et dermatologiques ; voir en particulier la demande WO 2005/044289), ceci est beaucoup moins le cas de S. *sphenanthera.* Plusieurs motifs expliquent cela :

- une moindre réputation quant à son utilisation selon la tradition chinoise,
- une quasi-absence d'usage en occident,
- une moindre teneur en néo-lignanes totaux comparée à S. *chinensis.*

Cependant, sur un plan commercial, le fruit de *S. sphenanthera* est considéré comme moins coûteux que celui de *S. chinensis.*

Le fruit de *S. sphenanthera* se caractérise par sa richesse en désoxyschisandrine. Par contre, ses teneurs en schisandrine et $\gamma$-schisandrine sont très faibles comparées à celles de la graine de *S. chinensis* (Zhu et al., 2007). Récemment, l'article (Huyke et al., 2007) a rapporté une étude comparative des effets sur la prolifération cellulaire d'extraits de *S. chinensis* et *S. sphenanthera.*

Le fruit sec de *Schizandra* comprend environ 20% d'huiles essentielles dont 7 à 30 % d'insaponifiables (Huyke et al., 2007). Les lignans sont compris dans la fraction insaponifiable des huiles. D'autres constituants actifs sont les phytostérols et les vitamines C et E.

#### *Huile **essentielle (monoterpènes et sesquiterpènes)***

L'huile essentielle est riche en dérivés sesquiterpéniques [Song et al., 2007] :

$\delta$-cadinène : 25,6 %,
$\gamma$-cadinène,
$\beta$-himachalène,
santalol.

L'huile essentielle du fruit de *S. chinensis* contient davantage d'hydrocarbures monoterpéniques que celle de

*S. sphenanthera* [Huyke et al., 2007].

***Stéroïdes***

Sitostérol [Huyke et al., 2007] (< 0,1 % dans le fruit)

***Alcool triterpénique***

schisanol (I) [Yue et al., 1994]

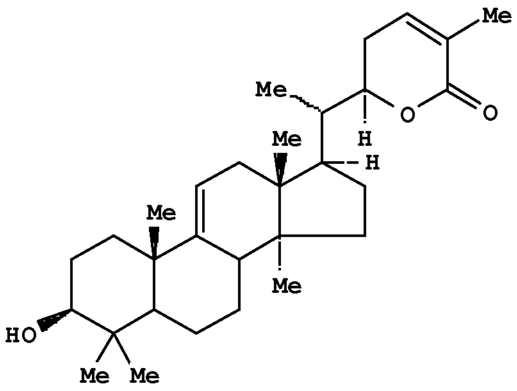

I

***Lignanes***

A. Données qualitatives

Les lignans (aussi appelés lignanes) sont considérés comme étant les principes actifs du fruit pour les applications dermatologiques, cosmétiques ou pharmaceutiques. Plus de 40 néo-lignanes à squelette dibenzo[a,c]cyclooctadiènes plus ou moins estérifiés par des acides organiques (acétique, benzoïque, angélique ou tiglique) ont été découverts dans des fruits des *Schisandra.* Il s'agit de constituants originaux. Ci-dessous une formule générale de lignane :

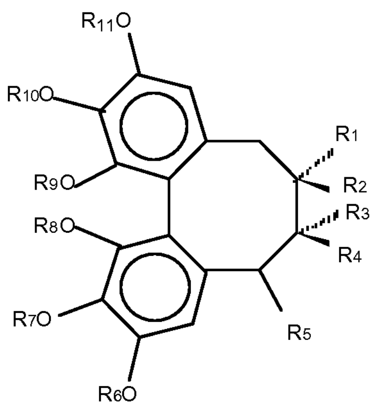

desoxyschisandrine : $R_{1,3,5}$ = H , $R_{2,4,6\text{-}11}$ = $CH_3$

La liste des néo-lignanes identifiés dans le fruit de *Schisandra sphenanthera* est présentée dans le tableau I. Pour les structures, voir les publications de Hancke et al., 1999 ; He et al., 1997 ; Huyke et al., 2007.

***Tableau I.***

| **Composés** | **Réf.** |
|---|---|
| *Majeurs* | |
| désoxyschisandrine (= wuweizisu A = schisandrine A) | (1, 2, 3) (très majoritaire) |
| schisanthérine C | (1) |
| « schisanthérine » pour Zhu et al. mais isomère de la gomisine C ! | (3) |
| *Mineurs* | |
| schisandrine (= schisandrol A) | (3) (sans doute des traces) |
| γ-schisandrine (= wuweizisu B = schisandrine B) | (1, 2, 3) (traces) |
| gomisine C (= schisanthérine A) | (1,4) |
| gomisine B (=schisanthérine B) | (1) |
| schisanthérine D | (1,4) |
| schisanthérine E | (1) |
| gomisine O | (2) |
| desangeloylgomisine B | (2) |
| benzoylgomisine U (I) | (5) |
| tigloylgomisine O (II) | (5) |
| gomisine U | (5) |
| epigomisine | (5) |
| benzoylgomisine Q (III) | (6) |
| benzoylgomisine P (IV) | (6) |
| angeloylgomisine P | (6) |
| tigloylgomisine P | (6) |
| (+)-gomisine K3 | (4) |
| (1) : Liu et al., 1978 ; (2) : Huyke et al., 2007 ; (3) : Zhu et al., 2007 ; (4) : Yue et al., 1994 ; (5) : Ikeya et al., 1991 ; (6) : Ikeya et al., 1990 | |

Un lignane de type diarylbutane dénommé Sphenanlignane (I) aurait été identifié dans la graine du fruit [Jiang et al., 2005], de structure suivante :

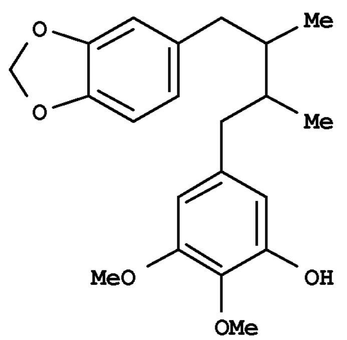

I

B. Données quantitatives

Des analyses effectuées par CCM-Densitométrie sur dix espèces de *Schizandra* auraient mis en évidence des différences de profils chromatographiques entre les espèces [Wang et al., 1991]. Il existe plusieurs données d'analyses des néo-lignanes de *S. sphenanthera* par CLHP, par exemple : Zhou et al., 2005.

Le tableau II (ci-après) présente les teneurs de néo-lignanes majeurs du fruit de S. *sphenanthera*, selon des articles publiés en anglais.

*Tableau II*

| **Composés** | **Teneurs (en mg/g)** | **dans le fruit sec** | **selon :** |
|---|---|---|---|
| Référence | Huyke et al., 2007 (*) | Zhu et al., 2007 | Wang et al., 2007 |
| désoxyschisandrine | 5.1 | 1,5-4,0 | 8,1-13,1 |
| "schisanthérine" (**) | | 0,8-3,5 | |
| schisanthérine C | | | 1,3-5,8 |
| gomisine O | 0,9 | | |
| désangéloylgomisine B | 0,6 | | |
| schisandrine | 0,1 | 0,06-0,67 | 0 |
| $\gamma$-schisandrine | 0,03 | 0 | 0 |
| (*) : résultats par extrapolation d'un extrait éthanolique<br>(**) : dénomination incomplète, structure isomère de la schisanthérine A | | | |

Le fruit de *S. sphenanthera* se caractérise par sa richesse en désoxyschisandrine. Par contre, ses teneurs en schisandrine et $\gamma$-schisandrine sont très faibles comparées à celles de la graine de *S. chinensis* (Zhu et al., 2007 ; ).

**Les extraits de fruit : formes utilisées**

A côté de son emploi traditionnel en Chine à l'état déshydraté, le fruit de *Schisandra sphenanthera* est aussi utilisé sous forme d'extraits obtenus par des solvants d'extraction permettant d'entraîner les néo-lignanes du fruit. Ces solvants mentionnés dans la littérature sont l'éthanol, le $CO_2$-supercritique, associé ou non avec un co-solvant, et l'hexane.

Plusieurs études publiées se sont attachées à comparer les pouvoirs extractifs du $CO_2$-SC, du chloroforme, du méthanol, de l'éthanol et l'éthanol à 50 % à l'égard des néo-lignanes du fruit.

Selon une publication récente, l'extraction du fruit de *S. sphenanthera* par le $CO_2$ ou le $CO_2$ + 5 % d'éthanol, ou encore par l'hexane, aboutit à des compositions d'extraits assez similaires au plan néo-lignanes. Par contre, l'utilisation d'éthanol a conduit à une moindre extraction de 2 néo-lignanes (la désydroschisandrine et la gomisine O), et apparemment une meilleure extraction de $\gamma$-schisandrine (Huyke et al., 2007).

Des conditions optimales d'extraction par $CO_2$-SC de néo-lignanes (desoxyschisandrine, $\gamma$-schisandrine ...) du fruit de *S. sphenanthera* ont été proposées dans l'article [Yang, 2001] : 21 MPa, 37°C, et un débit de 5 litres/min. de $CO_2$.

**Physiopathologie de l'erythro-couperose et de la rosacée**

La rosacée est une dermatose fréquente, affectant essentiellement le visage et se traduisant par des flushs (bouffées vasomotrices), un érythème permanent, des papules, des pustules et des télangectasies. Des critères secondaires peuvent souvent se manifester comme des sensations de brûlures, de picotements, des plaques rouges, une sécheresse cutanée, un oedème facial et phymas et des manifestations oculaires.

Cette dermatose peut être déclenchée et aggravée par de nombreux facteurs comme : les boissons chaudes, l'alcool, les changements de température, les aliments épicés, l'exercice, le stress et les fortes émotions, le soleil...

La physiopathologie de la rosacée a été longtemps définie comme mal connue et multifactorielle (facteur génétique, inflammatoire et vasculaire).

Récemment, il a été découvert et mis en évidence l'implication de différents mécanismes dans l'initiation de la rosacée (Yamasaki K. & Gallo RL., 2009) :

1) une immunité innée altérée : des variants des cathélicidines LL37 sont présents en quantité plus abondante que d'habitude ; de plus ces variants ont des propriétés inflammatoires et induisent la sécrétion d'IL-8.
2) des troubles vasculaires : une néoangiogenèse aboutissant à des télangectasies (petits vaisseaux apparents) ; sous l'effet de réactions vasomotrices répétées, se produisent des lésions du réseau lymphatique responsables d'une inflammation persistante.
3) l'action de microbes.
4) le stress oxydatif et le soleil.

Ces différents mécanismes sont liés entre eux, en particulier les cathélicidines pourraient être aussi déclencheurs des troubles vasculaires.

## ART ANTERIEUR

Il n'a pas été trouvé dans la littérature d'information concernant un usage industriel de *S. sphenanthera.* Il ne semble pas que le fruit de cette espèce soit exploité pour l'isolement de néo-lignanes, en dépit d'un brevet aux USA [US 5,484,595].

### *Usage médicinal:*

La demande WO 2007/020382 de la société Phynova décrit une composition comprenant des extraits de quatre plantes dont *Schisandra chinensis* ou *Schisandra sphenanthera*, pour le traitement de problèmes hépatiques, métaboliques et/ou immunitaires, et plus particulièrement destinée à traiter l'hépatite C.
Les demandes WO 2006/122485 et WO 2006/1222454 de la société Guang Zhou Zhongyi Pharmaceutical décrivent des compositions destinées à traiter le diabète, comprenant des mélanges de plusieurs plantes dont *Schizandra sphenanthera.*
La demande US 2003/0026854 de Mr Zhao décrit un médicament à base de schisandrine pour traiter les maladies du foie ; la schisandrine est en particulier extraite de *Schizandra chinensis* ou *Schizandra sphenanthera.*
La demande WO 2007/005760 décrit une composition comprenant des composés de la famille des schizandrines, gomisines, et autres composés issus d'extraits des fruits de *Schizandra chinensis* et *Schizandra sphenanthera*, pour traiter des cellules de cancer chimiorésistantes.
En Europe, aucun médicament autorisé par les Autorités Compétentes ne contient d'extrait de *Schisandra sphenanthera.* Il est généralement considéré que *S. sphenanthera* est inférieur à *S. chinensis* au plan médicinal et qu'il n'est utile qu'en tant que source alternative des lignans actifs.

### *Usage dermatologique et cosmétique :*

L'article (Huyke et al., 2007) décrit et compare les effets d'extraits de *Schizandra sphenanthera* et *S. chinensis* sur des cellules en culture : la prolifération des cellules épidermiques de types HaCaT et A431 est inhibée de manière dose-dépendante par ces extraits, les extraits non polaires étant plus efficaces que les extraits polaires. L'extrait au $CO_2$-SC de *Schisandra sphenanthera* s'est avéré le plus actif avec une $CI_{50}$ de 20 μg/ml. Dans un essai d'inhibition enzymatique avec cellules libres, l'extrait au $CO_2$-SC a fortement inhibé la production de prostaglandine par une cyclooxygénase-2 (COX-2 recombinée) ($CI_{50}$ = 0,2 μg/ml contre 1 μg/ml pour l'indométhacine). Il a également réduit la production de $PGE_2$ induite par les ultraviolets B ($CI_{50}$ = 4 μg/ml) et l'expression de COX-2 dans des kératinocytes HaCaT. Les auteurs de l'essai concluent que l'extrait au $CO_2$-SC de *Schisandra sphenanthera* pourrait être utile dans la prévention et le traitement des maladies inflammatoires et hyperprolifératives de la peau.
L'utilisation d'extraits de *Schizandra chinensis* dans la prévention de l'acné a été décrite dans la demande de brevet KR 2001931, dans des compositions comprenant en outre des agents d'exfoliation, choisis parmi des protéases de plantes telles que la papaïne et la bromelaïne, ou issues de micro-organismes.
Une composition anti-acnéique de médecine traditionnelle chinoise a été décrite dans la demande CN 11040971 : elle comprend 10 parts d'acore odorant appelé «shi chang pu », 8 parts de fruit de *Schizandra* et trois autres plantes.

## DESCRIPTION DE L'INVENTION

Les inventeurs ont découvert que les extraits du fruit de *Schizandra sphenanthera* présentent des propriétés cosmétiques et dermatologiques jamais décrites jusqu'à présent. En particulier, c'est la première fois que des extraits de *Schizandra sphenanthera* sont utilisés en tant que tels, pour leurs propriétés spécifiques, et non en alternative de *Schizandra chinensis.*
L'invention a pour objet une composition cosmétique, dermatologique ou nutraceutique telle que décrite dans la revendication 1, comprenant un extrait de fruit de *Schizandra sphenanthera*, éventuellement en association avec un excipient approprié.
Plusieurs extraits du fruit *Schizandra sphenanthera* peuvent être obtenus et utilisés, seuls ou en combinaison.
L'extrait de fruit de *Schizandra sphenanthera* est un extrait peptidique et osidique.
L'extrait de fruit de *Schizandra sphenanthera* est un extrait peptidique et osidique, ne comprenant pas de lignanes.
Par « extrait peptidique », on entend un extrait comprenant majoritairement des peptides. Par « extrait osidique » on entend un extrait comprenant majoritairement des oses. L'extrait peptidique selon l'invention présente avantageusement les spécifications suivantes :

| | |
|---|---|
| Teneur en peptides (%) | 5 - 90 |
| Teneur en sucres totaux (%) | 5 - 90 |

Selon un aspect préféré de l'invention, l'extrait peptidique et osidique est constitué de 10 à 50% en poids de peptides et de 10 à 60% en poids de sucres, les pourcentages étant exprimés par rapport au poids total dudit extrait.

L'invention a également pour objet un procédé de préparation d'un extrait peptidique et osidique de fruit de *Schizandra sphenanthera.* Ce procédé de préparation d'un extrait peptidique et osidique de fruit de *Schizandra sphenanthera* comprend les étapes successives suivantes :

a) à partir de fruit de *Schizandra,* extraction d'une huile brute et d'un tourteau et récupération dudit tourteau qui est dispersé dans de l'eau ;
b) traitement enzymatique dudit tourteau par un mélange enzymatique de cellulases, protéases, alpha-amylases, puis ;
c) traitement thermique afin d'inhiber les enzymes ;
d) centrifugation, ultrafiltration et diafiltration sur une membrane ayant un seuil de coupure de 15 kDa afin d'éliminer les protéines résiduelles ;
e) nanofiltration afin d'éliminer des sels minéraux ou des acides aminés libres par exemple ;
f) l'extrait peptidique peut être décoloré en présence de charbon actif puis il est récupéré après filtration.

Avantageusement, l'extrait peptidique et osidique peut être lyophilisé.

Selon un aspect de l'invention, la composition cosmétique, dermatologique ou nutraceutique comprend au moins deux extraits de fruit de *Schizandra Sphenanthera.* En particulier la composition comprend un extrait peptidique et osidique et une huile brute ; ou un extrait peptidique et osidique et une huile raffinée ; ou un extrait peptidique et osidique et un concentrat d'huile raffinée ; ou un extrait peptidique et osidique et un insaponifiable.

Les extraits lipidiques (huile brute, huile raffinée) peuvent être obtenus par plusieurs procédés :

- extraction physique telle que la pression à froid sur presse mécanique, pression sur extrudeuse bi-vis, ...
- extraction chimique à l'aide de solvants organiques (alcanes aliphatiques, alcools, solvants chlorés, solvants fluorés, ...)
- extraction en milieu supercritique, à l'aide du dioxyde de carbone seul et/ou avec co-solvants.

Il sera préféré, pour l'extraction d'huile de *Schizandra sphenanthera*, d'utiliser un solvant en milieu supercritique tel que le dioxyde de carbone.

L'huile brute de *Schizandra sphenanthera* est raffinée selon un procédé de distillation moléculaire afin d'éliminer la fraction terpénique. L'huile raffinée est donc « une huile brute sans terpènes ».

Par « concentrat », on entend « huile raffinée ayant subi une étape de distillation moléculaire ».

Le terme « insaponifiable » désigne la fraction insaponifiable du concentrat d'huile raffinée, obtenue après une étape de saponification, et une étape d'extraction de l'insaponifiable grâce à un solvant approprié.

La préparation d'un concentrat d'huile raffinée de fruit de *Schizandra sphenanthera*, concentrée en sa fraction insaponifiable est décrite. Ce procédé comprend une étape de distillation moléculaire d'une huile raffinée. En particulier, l'étape de distillation moléculaire est réalisée en utilisant un dispositif choisi parmi les distillateurs moléculaires de type centrifuge et les dispositifs moléculaires de type à film raclé tels que décrits précédemment.

Un procédé de préparation d'un insaponifiable de fruit de *Schizandra sphenanthera* est décrit. Il comprend une étape de saponification d'un concentrat d'huile raffinée de fruit de *Schizandra sphenanthera*, puis une extraction de cet insaponifiable à l'aide d'un solvant approprié. Cet extrait est ensuite lavé jusqu'à élimination complète des savons puis le solvant est évaporé. Enfin l'insaponifiable subit une désodorisation à la vapeur d'eau suivi d'un stripping à l'azote afin d'éliminer les traces de solvant et d'eau.

Selon un autre aspect de l'invention, la composition cosmétique, dermatologique ou nutraceutique peut en outre comprendre au moins un autre composé actif en plus de l'extrait de fruit de *Schizandra sphenanthera.*

Cet autre composé peut être choisi parmi tous les composés et leurs équivalents fonctionnels, énoncés ci-dessous.

Cet autre composé peut être en particulier choisi parmi des actifs classiquement utilisés en dermatologie tels que les émollients, les actifs hydratants, les activateurs de la synthèse de kératine, les kératorégulateurs, les kératolytiques, les agents restructurant de la barrière cutanée (activateurs de la synthèse des lipides cutanés), des agonistes PPARs (ou Peroxysome Proliferator Activated Receptor), les agonistes RXR ou LXR, les agonistes des récepteurs à

la vitamine D ou aux corticoïdes, les activateurs de la différenciation des kératinocytes (rétinoïdes, calcidone®, le calcium), les agents sébo-régulateurs, les agents anti-irritants, les agents apaisants, les agents anti-inflammatoires, les agents anti-oxydants et les agents anti-âge.

Cet autre composé peut aussi être choisi parmi des principes actifs ayant une action thérapeutique complémentaire, tels que les antibiotiques, les pré et probiotiques, les agents anti-bactériens, les composés antifongiques, les agents anti-viraux, les conservateurs, les immuno-modulateurs (tacrolimus ou pimécrolimus), les oxazolines, les facteurs de croissance, les agents cicatrisants ou les molécules eutrophiques, les médicaments, les agents pigmentants ou hypopigmentants, les agents lipolytiques ou inhibiteurs de la lipogénèse ou encore les agents anti-cellulite ou amincissants, les filtres et écrans solaires minéraux ou organiques (pigmentaires ou ultrafins), des aliments classiques ou fonctionnels : hyper ou hypoglycémiants, des nutriments anti-graisse ou anti-cellulite, anti-cholestérol, anti-oxydant, énergisant, reconstituant, ayant un impact sur les signes secondaires de la ménopause.

Cet autre composé peut aussi être choisi parmi des extraits naturels de plante (parties de végétaux extractibles en phase aqueuse ou huileuse : polyphénols, flavonoïdes, autres peptides et sucres, .....), des composés contenant des insaponifiables d'huiles végétales, des insaponifiables stéroliques ou des produits pouvant en contenir (insaponifiables d'huiles végétales, notamment insaponifiables d'huile de Soja, insaponifiables de beurres végétaux ou de matières butyreuses et leurs mélanges, insaponifiables de cires naturelles, insaponifiables d'extraits huileux, insaponifiables de co-produits huileux industriels, insaponifiables d'extraits de corps gras d'origine animale, insaponifiables d'huiles marines, insaponifiables d'extraits de la matière grasse lactique, insaponifiables de lipides extraits d'organismes unicellulaires, insaponifiables des lipides extraits des algues et organismes marins, etc), des stérols, des stanols, des phytostérols, des phytostanols, des tocophérols, des concentrats d'huiles de Tournesol, de Colza et/ou de Palme, des oligo-éléments, des vitamines, des acides gras en oméga 3, 6 ou 9, des plantes hypoglycémiantes ou hyperglycémiantes ou sucrantes.

Les actifs hydratants/émollients les plus utilisés sont la glycérine ou ses dérivés, l'urée, l'acide pyrrolidone carboxylique et ses dérivés, l'acide hyaluronique de tout poids moléculaire, les glycosaminoglycanes et tout autres polysaccharides d'origine marine, végétale ou biotechnologique comme par exemple la gomme xanthane, le fucogel®, certains acides gras comme l'acide laurique, l'acide myristique, les acides gras poly- et mono-insaturés de type oméga 3, 6 et 7, 9 comme l'acide linoléique et acide palmitoléique, l'oléodistillat de Tournesol, les peptides d'Avocat, le beurre de Cupuaçu.

Les activateurs de la synthèse de kératine pouvant être utilisés en association sont avantageusement les rétinoïdes, les peptides de Lupin, des protéines clés du *stratum corneum* ou *granulosum* (kératines) et des cornéodesmosomes, les sucres d'Avocat, un extrait peptidique de Quinoa.

Les agents anti-inflammatoires / anti-irritants, apaisants limitent la réaction inflammatoire conduite *via* les cytokines ou médiateurs du métabolisme de l'acide arachidonique et ont des propriétés apaisantes et anti-irritantes. Les plus classiques sont l'acide glycyrrhétinique (les dérivés de réglisse) avec ses sels et esters, l'acide lipoïque, le beta-carotène, la vitamine B3 (niacinamide, nicotinamide), la vitamine E, la vitamine C, la vitamine B12, les flavonoïdes (thé vert, quercétine...), le lycopène ou la lutéine, les sucres d'Avocat, l'oléodistillat d'Avocat, l'arabinogalactane, les peptides de Lupin, un extrait total de Lupin, un extrait peptidique de Quinoa, le Cyclocéramide® (dérivé d'oxazoline), les isoflavones comme par exemple la génistéine/génistine, daidzéine/daidzine, les eaux de source ou thermales (eau d'Avène, eau de la Roche Posay, eau de Saint Gervais, eau d'Uriage, eau de Gamarde), les extraits de Goji (*Lycium barbarum*), les peptides ou complexes d'acides aminés végétaux ou encore. la disulone topique, ou les médicaments anti-inflammatoires stéroïdiens (AIS), tels que les corticoïdes, ou non-stéroïdiens (AINS).

Parmi les agents kératorégulateurs/kératolytiques les plus utilisés se trouvent : les acides de fruits alpha hydroxyacide - AHA (acide citrique, acide glycolique, acide malique, acide lactique...), les esters d'AHA, les associations d'AHA avec d'autres molécules comme l'association acide malique et protéines d'amandes (keratolite®), l'association acide glycolique ou acide lactique avec l'arginine ou encore l'association d'hydoxy-acide avec des molécules lipidiques comme le LHA® pour lipo-hydroxy-acide, les complexes d'hydroxyacide amphotères - AHCare, l'ecorce de saule (Salix alba bark extract), l'acide azélaïque et ses sels et esters, l'acide salicylique et ses dérivés comme l'acide capriloyl salicylique ou en association avec d'autres molécules comme l'association acide salicylique et polysaccharide (beta hydroxyacide - BHA), le tazarotène, l'adapalène, ainsi que les molécules de la famille des rétinoïdes tels que : la trétinoïne, le rétinaldéhyde, l'isotrétinoïne, le rétinol.

Les agents sébo-régulateurs pouvant être utilisés en association sont avantageusement choisis dans le groupe constitué par les inhibiteurs de 5-alpha réductase comme par exemple l'actif 5-alpha Avocuta®. Le zinc (et ses sels gluconate, salicylate et acide pyroglutamique) présente aussi une activité sébo-suppresseur. On peut citer aussi la spironolactone, anti-androgène et antagoniste de l'aldostérone, qui engendre une réduction significative du taux de sécrétion de sébum après douze semaines d'application. D'autres molécules telles que par exemple *Cucurbita pepo,* extraite des graines de citrouille, et l'huile de pépins de courge, le sabal, limitent la production de sébum par inhibition de la transcription et de l'activité de la 5$\alpha$-réductase. D'autres séborégulateurs d'origine lipidique agissant sur la qualité du sébum, comme l'acide linoléique présentent un intérêt.

Les facteurs de croissance pouvant être utilisés en association sont avantageusement la becaplermine et le

TGF-beta (Transforming Growth Factor beta), l'EGF, le NGF, le VEGF.

On entend par agent antioxydant, une molécule qui diminue ou empêche l'oxydation d'autres substances chimiques. Les antioxydants pouvant être utilisés en association sont avantageusement choisis dans le groupe constitué par les thiols et les phénols, parmi les dérivés de réglisse comme l'acide glycyrrhétinique avec ses sels et esters, l'alpha bisabolol, l'extrait de ginkgo biloba, de calendula, le Cyclocéramide® (dérivé d'oxazoline), les peptides d'Avocat, les oligo-éléments comme le cuivre, le zinc, et le sélénium, l'acide lipoïque, la vitamine B12, la vitamine B3 (niacinamide, nicotinamide), les vitamines C, les vitamines E, le co-enzyme Q10, le krill, le glutathion, le BHT pour butylhydroxytoluène, le BHA pour butylhydroxyanisol, le lycopène ou la lutéine, le beta-carotène, la grande famille des polyphénols comme les tanins, les acides phénoliques, les anthocyanes, les flavonoïdes avec par exemple les extraits de thé vert, de fruits rouges, de cacao, de raisin, de *Passiflora incarnata*, de *Citrus* ou encore les isoflavones comme par exemple la génistéine/génistine, daidzéine/daidzine, Dans le groupe des anti-oxydants on trouve aussi les substances anti-glycation telles que la carnosine ou certains peptides, la n-acetyl-cystéine, ainsi que les enzymes antioxydants ou antiradicalaires comme la SOD (super oxyde dismutase), la catalase, la glutathion peroxydase, la thioredoxine reductase et leurs agonistes.

Les agents cicatrisants et restructurant de la barrière cutanée, permettant de stimuler la synthèse des lipides clés de l'épiderme, et pouvant être utilisés en association, sont avantageusement la vitamine A, le panthénol (vitamine B5), les sucres d'Avocat, le Lupéol, l'extrait peptidique de Maca, un extrait peptidique de Quinoa, l'arabinogalactane, l'oxyde zinc, le magnésium, le silicium, l'acide madécassique ou asiatique, le sulfate de dextran, le coenzyme Q10, la glucosamine et ses dérivés, la chondroïtine sulfate et globalement les glucosaminoglycanes ou GAG, le sulfate de dextran, les céramides, le cholestérol, le squalane, les phospholipides, les peptides de soja fermenté ou non, les peptides végétaux, les polysaccharides marins, végétaux ou biotechnologiques comme les extraits d'algues ou l'extrait de fougère, les oligo-éléments, les extraits de plantes à tanin comme les tanins dérivant de l'acide gallique appelés tanins galliques ou encore hydrolysables, initialement trouvés dans la noix de galle, et les tanins catéchiques résultant de la polymérisation d'unités flavaniques dont le modèle est fourni par le Cachou (*Acacia catechu*).

Les oligo-éléments pouvant être utilisés sont avantageusement choisis dans le groupe constitué par le cuivre, le magnésium, le manganèse, le chrome, le sélénium, le silicium, le zinc et leurs mélanges. Peuvent également être utilisés des concentrats de Tournesol, plus avantageusement des concentrats de Tournesol linoléiques, tels que l'actif commercialisé par les Laboratoires Expanscience, Soline®, des insaponifiables d'huile végétale, tel que l'Avocadofurane®, des agonistes PPARs (rosiglitazone, pioglitazone), RXR, LXR.

Les agents anti-âge pouvant agir en association pour la prise en charge de l'acné sur les sujets d'âge mûr sont des agents antioxydants et en particulier la vitamine C, la vitamine A, le rétinol, le rétinal, l'acide hyaluronique de tout poids moléculaire, l'Avocadofurane®, les peptides de Lupin, l'extrait peptidique de Maca.

Les composés antifongiques pouvant être utilisés en association sont avantageusement l'econazole et le ketoconazole.

Les conservateurs antiseptiques pouvant être utilisés en association sont par exemple le triclosan, la chlorhéxidine, les ammoniums quaternaires.

Les antibiotiques pouvant être utilisés en association sont avantageusement l'acide fucidique, la pénicilline, les tétracyclines, la pristinamycine, l'érythromycine, la clindamycine, la mupirocine, la minocycline, la doxycycline. Les agents anti-viraux pouvant être utilisés en association sont avantageusement l'acyclovir et le valacyclovir.

Les conservateurs pouvant être utilisés en association sont par exemple ceux généralement utilisés en cosmétique ou en nutraceutique, les molécules à activité anti-bactérienne (pseudo-conservateurs) tels que les dérivés capryliques comme par exemple le capryloyl glycine et le glyceryl caprylate, tels que l'hexanediol, et le sodium levulinate, les dérivés de zinc et de cuivre (gluconate et PCA), la phytosphingosine et ses dérivés, le peroxyde de benzoyle, la piroctone olamine, le pyrithione zinc, le sulfure de sélénium.

Les actifs protecteurs solaires, tels que des filtres ou écrans solaires UVB et/ou UVA ; tels les écrans ou filtres minéraux et/ou organiques connus de l'homme du métier qui adaptera leur choix et leurs concentrations en fonction du degré de protection recherché.

A titre d'exemple d'actifs protecteur solaire, on peut notamment citer le dioxyde de titane, l'oxyde de zinc, le méthylène bis-benzotriazolyl tétraméthylbutylphénol (nom commercial : TINOSORB M) et le Bis-éthylhéxyloxyphénol méthoxyphényl triazine (nom commercial : TINOSORB S), l'octocrylène, le butyl méthoxydibenzoylméthane, l'acide terephthalylidene dicamphor sulfonique, le 4-méthylbenzylidène de camphre, le benzophénone, l'éthylhexyl méthoxycinnamate, l'éthylhexyl diméthyl PABA, le diéthylhexyl butamido triazone.

Les agents amincissants pouvant être utilisés en association sont avantageusement la caféine, le fucus, les extraits végétaux comme par exemple : l'extrait de lierre, de cacao, de guarana, de petit houx, de thé vert, de maté, de poivre de sichuan, de marron d'inde. La *Centella asiatica,* la carnithine, la glauscine, l'escine, l'extrait de petit houx (*ruscus*), les isoflavones comme par exemple la génistéine, le ginko biloba, la forskoline, le rétinol et autres rétinoïdes, la phloridzine, la criste marine.

Les agents anti-chute des cheveux et/ou fortifiant pour le cheveu et les ongles sont avantageusement les

phytostérols, les isoflavones comme par exemple les isoflavones de soja, le RTH16®, l'aminexil®, le minoxidil®, le rétinol, le zinc et ses dérivés, la neoruscine, la vitamine E, la vitamine B2, la vitamine B3, la vitamine B6, la vitamine PP, la vitamine B5 (panthénol, bépanthène), la vitamine B8 (vitamine H ou biotine), la vitamine B9 (acide folique), l'alpha hydroxyacide, la quinine, certains acides aminés comme la cystéine, la cystine, la méthionine. Peuvent également être utilisés les inhibiteurs de 5-alpha réductase tels que par exemple le finastéride, le dutastéride, *serenoa serrulata* ou *repens*, l'extrait de *Cucurbita pepo* ou certains phytostérols. On citera également la kératine, les oligoéléments, les sels minéraux, certains extraits protéiques ou lipidiques végétaux comme par exemple les extraits de pfaffia, de sauge, de citron, de ginseng, de quinquina, de jojoba, de marron d'inde, de miel, de blé, d'ortie, d'échinea, ou de noix de coco.

Les agents anti-pelliculaires (pour le cuir chevelu) sont avantageusement choisis parmi l'extrait de capucine, la vitamine F, le thymol, l'argile, le pyrithione de zinc, le zinc-PCA, le gluconate de zinc, le sulfate de zinc, le camphre, l'extrait de myrte, l'acide salicylique, la vitamine B5, le climbazole, l'ichtyol, le sélénium et ses dérivés, l'extrait de Curbicia, l'extrait de Carthame, l'extrait d'huile de Melaleuca, l'huile de Bourrache et de Mimosa Tenuiflora, la propolis, Kertyol, l'acide glycolique, le kéluamid, la cyclopiroxolamine. le piroctone olamine, le capryloyl glycine, et le 5 alpha Avocuta.

Les médicaments ou agents cosmétiques pouvant être utilisés en association sont avantageusement les médicaments appropriés pour la prévention et/ou le traitement de :

- l'atopie :les corticostéroïdes comme l'hydrocortisone, le desonide, l'acetonide de fluocinolone, le propionate de fluticasone, les immunomodulateurs topiques inhibiteurs de la calcineurine comme le tacrolimus et le pimecrolimus, la cyclosporine, l'azathioprine, le méthotrexate, la vitamine B12, les molécules anti-microbiennes, les anti-histaminiques comme l'hydroxyzine et la diphenhydramine, les antibiotiques, les pro-biotiques, la naltrexone, les agonistes de PPAR alpha tels que l'oléodistillat de Tournesol, les émollients contenant des céramides ou autres lipides clés épidermiques,
- de l'acné : les antibiotiques, le péroxyde de benzoyle, les rétinoïdes, l'acide azélaïque, la vitamine PP, la vitamine B3, le zinc, les cyclines,
- de l'eczéma : les immuno-modulateurs, les émollients, l'huile de saumon, de bourrache, les pré-biotiques
- de la rosacée : le perméthol, la génistéine, l'esculoside, le sulfate de dextran, l'hespéridine méthylchalcone, les rétinoïdes, la licochalcone, l'oxyméthazoline, la kinétine, l'extrait de réglisse, les polyphénols, les flavonoïdes, les procyanidols (thé vert), la vitamine P like, l'extrait de petit houx, le S*ophora japonica,* l'extrait d'Hamamélis, les antibiotiques tes que la doxycycline,
- du psoriasis : les corticoïdes, le calcipotriol, le calcitriol, le tazarotène, l'huile de cade, l'acitrétine, la PUVA thérapie.

Les médicaments ou aliments pouvant être utilisés en association sont avantageusement des médicaments ou aliments hyperlipidémiants et/ou hypolipidémiants. On citera notamment les médicaments à base de sulfonylurées et de glinides, les médicaments à base d'inhibiteurs des alpha-glucosidases, les médicaments à base de biguanides (metformine), les médicaments à base d'activateurs de la sensibilité à l'insuline ou thiazolidinediones (TZD, pioglitazone, rosiglitazone), qui sont des agonistes PPARs, les médicaments hypolipidémiants de la famille des statines ou de la famille des fibrates (agonistes de PPAR$\alpha$), l'orlistat (Xenical) et la sibutramine (Réductyl ou Sibutral).

Les nutriments anti-graisse pouvant être utilisés en association sont avantageusement choisis dans le groupe constitué par des nutriments bloqueurs de l'absorption des graisses, tels que le chitosan, des nutriments capables d'augmenter la thermogénèse (« brûleur de graisse ») tels que l'éphédrine (herbe chinoise Ma Huang), la caféine, la théophylline et théobromine et le *Citrus aurantium*, des nutriments capables de réguler l'appétit (« coupe faim ») tels que la L-phénylalanine et la L-tyrosine, des nutriments capables de réguler la glycémie, tels que des minéraux, par exemple le chrome ou le vanadium ou le magnésium, ou l'herbe ayurvédique Gymnema sylvestre, des inhibiteurs de la lipogénèse, tel que l'acide hydroxycitrique extrait du Garcinia cambodgia et des nutriments capables de transporter des graisses tels que la L-carnitine.

Des exemples d'aliments ou de thérapies hyperglycémiantes, pour rééquilibrer la glycémie, sont les antirétrovirus, les glucocorticoïdes, les immunosuppresseurs, IFN-Alpha, les stéroïdes sexuels, le THS, la pilule, les hormones de croissance, les sympathomimétiques, les médicaments cardio-vasculaires, les diurétiques, les Bêtabloquants, les inhibiteurs calciques, les psychotropes.

Les plantes hypoglycémiantes pouvant être utilisés en association sont avantageusement choisis dans le groupe constitué par le fenugrec (*Trigonella graenum*), l'acide corosolique (composé actif des feuilles de l'arbre *Lagestroemia speciosa*), le *Gymnema syllvestre,* le jus de fruit de momordique (*Momormodica charantia*), l'eucalyptus (*Eucalyptus globulus),* le *Panax ginseng,* les feuilles de myrtille (*Vaccinum myrtillus*).

Les immnumodulateurs pouvant être utilisés en association sont avantageusement le tacrolimus, le pimécrolimus et les oxazolines. Les oxazolines pouvant être utilisées en association sont avantageusement des oxazolines choisies dans le groupe constitué par la 2-undécyl-4-hydroxyméthyl-4-méthyl-1,3-oxazoline, la 2-undécyl-4,4-diméthyl-1,3-oxazoline, la (E)-4,4-diméthyl-2-heptadéc-8-ényl-1,3-oxazoline, la 4-hydroxyméthyl-4-méthyl-2-heptadécyl-1,3-oxazoline, la (E)-4-hydroxyméthyl-4-méthyl-2-heptadéc-8-ényl-1,3-oxazoline, la 2-undécyl-4-éthyl-4-hydroxyméthyl-1,3-oxazoline. De manière encore plus avantageuse, ladite oxazoline est la 2-undécyl-4,4-diméthyl-1,3-oxazoline, appelée OX-100 ou Cyclocéramide®.

Les agents hypopigmentants ou dépigmentants pouvant être utilisés en association sont l'hydroquinone et ses dérivés, l'arbutine, l'acide rétinoïque, le rétinol, le rétinaldéhyde, la trétinoine, l'hydroquinone, les corticoïdes, l'acide kojique, l'acide azélaïque, l'acide ellagique, l'acide pyruvique, l'acide glycolique, la vitamine B3 (niacinamide) ou PP, la vitamine C, le Cyclocéramide®, les dérivés du résorcinol, le résvératrol, des extraits de réglisse ou de murier blanc, l'acide alpha-lipoïque, l'acide linoléique, l'indométhacine, des chélateurs de cations tels que l'EDTA (acide éthylène diamine tétra acétique), les extraits de soja tels que la génistéine. On peut également citer le Sepiwhite® (N-undecylenoyl-L-phénylalanine) commercialisé par la société Seppic, qui est un agent cosmétique dépigmentant.

Les agents pigmentants pouvant être utilisés en association sont par exemple des agents qui colorent la peau comme le dihydroxyacétone et les mélanines ; des agents qui stimulent le procédé de pigmentation naturelle comme les psolarènes ayant des propriétés thérapeutiques en dermatologie (le 8-méthoxypsolarène, le 5-méthoxypsolarène, le 4,5',8-triméthylpsolarène ou des extraits végétaux de *Psorelea corylifolia* et de *Ammi majus*), les caroténoïdes (le lycopène, la canthaxanthine), les agents stimulant la voie de l'AMP cyclique (1. les analogues de l'AMPc, tels que le 8-bromo-AMPc ou le dibutiryl-AMPc, 2. la forskoline, 3. l'isobutyl-méthyl-xanthine ou la théophylline), les activateurs des protéines kinase C (les diacylglycérols, en particulier l'oléyl-acétyl-glycérol), diols aliphatiques ou cycliques (le 1,2-propandiol, le 5-norbomane-2,2-diméthanol, le norbomane-2,2-diméthano), les diols bicycliques monoterpène, les dérivés de tyrosine (la L-tyrosine, la L-DOPA), le diméthylsulfoxyde, les agents lysomotropiques, les dinucléotides thymidine, les fragments d'ADN, les analogues de l'hormone stimulant les mélanocytes, le 3-isobutyl-1-méthylxanthine, les donneurs d'acide nitrique (Brown, Journal of photochemistry and photobiology B :biology 63 (2001) 148-161) ; ou encore des extraits végétaux comme les peptides de riz, et les algues, démontrant une activité pro-mélanogène : *Laminaria digitata* (Thalitan® de Codif).

Une association particulièrement avantageuse selon l'invention est une composition comprenant l'extrait de fruit peptidique et osidique de *Schizandra sphenanthera* et des insaponifiables végétaux et animaux, comme par exemple, les insaponifiables d'avocat et/ou de soja, et des concentrats d'huile végétale ou animale en insaponifiable, comme par exemple le concentrat d'huile de Tournesol ou d'huile de Palme, ou encore des huiles végétales contenant des insaponifiables comme par exemple les huiles de soja et de colza, et les dérivés d'insaponifiable comme les furanes d'avocat, les esters de stérols et les dérivés vitaminiques. Les insaponifiables « stéroliques » sont des insaponifiables dont la teneur en stérols, en méthylstérols et en alcools triterpèniques est comprise entre 20 et 95 % en poids, de préférence 45-65 % en poids, par rapport au poids total de l'insaponifiable.

Une association particulièrement avantageuse selon l'invention est une composition comprenant l'extrait de fruit de *Schizandra sphenanthera* et des sucres d'avocat (tels que décrits dans la demande WO 2005/115421). Cette composition est particulièrement adaptée pour le traitement de la réparation de la barrière cutanée et de l'inflammation.

Une association particulièrement avantageuse selon l'invention est une composition comprenant l'extrait de fruit de *Schizandra sphenanthera* et des peptides d'avocat (tels que décrits dans la demande internationale WO 2005/105123). Cette composition est particulièrement adaptée pour le traitement de l'irritation et de l'inflammation.

Une autre association particulièrement avantageuse selon l'invention est une composition comprenant l'extrait de fruit de *Schizandra sphenanthera* et une huile d'avocat (telle que décrite dans les demandes internationales WO 2004/012496, WO 2004/012752, WO 2004/016106, WO 2007/057439).

Une autre association particulièrement avantageuse selon l'invention est une composition comprenant l'extrait de fruit de *Schizandra sphenanthera* et l'Avocadofurane® (furanes d'avocat, pouvant être obtenus par le procédé décrit dans la demande internationale WO 01/21605). Cette composition est particulièrement adaptée pour le traitement de l'inflammation, pour favoriser la cicatrisation, et pour ses propriétés anti-âge.

Une autre association particulièrement avantageuse selon l'invention est une composition comprenant l'extrait de fruit de *Schizandra sphenanthera* et le 5 alpha Avocuta® (butyl avocadate). Cette composition est particulièrement adaptée pour inhiber la 5-alpha réductase (voir WO 01/52837 et WO 02/06205) et réguler la sécrétion séborrhée se trouvant augmentée dans l'acné ou les pellicules.

Une autre association particulièrement avantageuse selon l'invention est une composition comprenant l'extrait de fruit de *Schizandra sphenanthera* et les insaponifiables d'avocat et de soja pouvant être utilisés en association sont avantageusement un mélange d'insaponifiables d'Avocat furanique et d'insaponifiables de soja, dans un rapport respectif d'environ 1/3-2/3. Les insaponifiables d'avocat et de soja sont encore plus avantageusement le produit Piasclédine®, commercialisé par les Laboratoires Expanscience.

Une autre association particulièrement avantageuse selon l'invention est une composition comprenant l'extrait de fruit de *Schizandra sphenanthera* et un oléodistillat de tournesol, encore plus avantageusement avec des concentrats de Tournesol linoléiques, tels que l'actif commercialisé par les Laboratoires Expanscience, Soline® (cf. la demande internationale WO 01/21150), Cette composition est particulièrement adaptée pour le traitement de l'inflammation et pour la réparation de la barrière cutanée.

Une autre association particulièrement avantageuse selon l'invention est une composition comprenant l'extrait

de fruit de *Schizandra sphenanthera* et un insaponifiable de Soja, tel qu'obtenu selon le procédé décrit dans la demande internationale WO 01/51596.

Une autre association particulièrement avantageuse selon l'invention est une composition comprenant l'extrait de fruit de *Schizandra sphenanthera* et du Lupéol (FR 2 822 821, FR 2 857 596). Cette composition est particulièrement adaptée pour favoriser la cicatrisation.

Une autre association particulièrement avantageuse selon l'invention est une composition comprenant l'extrait de fruit de *Schizandra sphenanthera* et des peptides de Lupin tels qu'obtenus selon le procédé décrit dans la demande WO2005/102259. Cette composition est particulièrement adaptée pour le traitement de l'inflammation et est utilisée pour ses propriétés anti-âge.

Une autre association particulièrement avantageuse selon l'invention est une composition comprenant l'extrait de fruit de *Schizandra sphenanthera* et un extrait total de Lupin (voir demande internationale WO 2005/102259). Cette composition est particulièrement adaptée pour le traitement des irritations.

Une autre association particulièrement avantageuse selon l'invention est une composition comprenant l'extrait de fruit de *Schizandra sphenanthera* et une huile de Lupin, avantageusement une huile de Lupin blanc doux, telle que celle décrite dans la demande internationale WO 98/47479.

Une autre association particulièrement avantageuse selon l'invention est une composition comprenant l'extrait de fruit de *Schizandra sphenanthera* et un extrait peptidique de Maca (voir demande internationale WO2004/112742). Cette composition est particulièrement appréciée pour ses propriétés cicatrisantes et anti-âge.

Une association particulièrement avantageuse selon l'invention est une composition comprenant l'extrait de fruit peptidique et osidique de *Schizandra sphenanthera* et des peptides de Riz (voir demande internationale WO 2008/009709). Cette composition est particulièrement appréciée pour ses propriétés de stimulation de la mélanogénèse et du transfert de la mélanine.

Une autre association particulièrement avantageuse selon l'invention est une composition comprenant l'extrait de fruit de *Schizandra sphenanthera* et du Cyclocéramide® (dérivé d'oxazoline) tel que décrit dans les demandes internationales WO 2004050052, WO 2004050079, et WO 2004112741. Cette composition est particulièrement adaptée pour le traitement des réactions inflammatoires.

Une autre association particulièrement avantageuse selon l'invention est une composition comprenant l'extrait de fruit de *Schizandra sphenanthera* et un extrait de Quinoa, en particulier un extrait peptidique (voir la demande internationale WO 2008/080974). Cette composition est particulièrement adaptée pour le traitement des conditions inflammatoires et de la réparation de la barrière cutanée.

Une autre association particulièrement avantageuse selon l'invention est une composition comprenant l'extrait de fruit de *Schizandra sphenanthera* et un beurre de Cupuaçu. Cette composition est particulièrement appréciée pour ses propriétés hydratantes.

Une autre association particulièrement avantageuse selon l'invention est une composition comprenant l'extrait de fruit de *Schizandra sphenanthera* et un concentrat de Colza.

Une autre association avantageuse selon l'invention est une composition comprenant l'extrait de fruit de *Schizandra sphenanthera* et un concentrat de Maïs.

Toutes ces associations comprennent au moins un autre composé actif, en plus de l'extrait de fruit de *Schizandra sphenanthera*, et peuvent comprendre deux, trois, quatre ou plus de composés actifs tels que décrits précédemment.

La composition selon l'invention peut être formulée sous la forme de différentes préparations adaptées à une administration topique, à une administration orale, rectale, vaginale, nasale, auriculaire ou bronchique, à une administration parentérale.

Selon une première variante, les différentes préparations sont adaptées à l'administration topique et incluent les crèmes, les émulsions, les laits, les pommades, les lotions, les huiles, les solutions aqueuses ou hydro-alcooliques ou glycoliques, les poudres, les patchs, les sprays ou tout autre produit pour application externe.

Selon une deuxième variante, les différentes préparations sont adaptées à une administration orale ; l'extrait de fruit de *Schizandra sphenanthera* pouvant entrer soit dans une composition alimentaire soit dans un complément alimentaire. Le complément alimentaire peut se présenter sous forme de l'extrait de fruit de *Schizandra sphenanthera* en tant que tel ou bien sous forme de gélules ou de capsules molles de gélatine ou végétales dans le cadre de la présente invention. Ledit complément alimentaire peut alors contenir de 10 à 100% en poids de l'extrait de fruit de *Schizandra sphenanthera.*

Selon cette deuxième variante de la présente invention, on peut incorporer, sans aucune restriction, les extraits de fruit de *Schizandra sphenanthera* de la présente invention dans la nourriture, les boissons et les nutraceutiques, y compris dans ceux cités en exemple ci-dessous :

1) <u>Les produits laitiers :</u> tels que les fromages, le beurre, le lait et autres breuvages lactés, mélanges et pâtes à tartiner à base de produits lactés, crèmes glacées et yaourts ;
2) <u>Les produits à base de graisse</u> tels que les margarines, pâtes à tartiner, mayonnaises, matières grasses pour

cuisson, huiles à frire et vinaigrettes ;

3) Les produits à base de céréales composés de graines tels que le pain et les pâtes, que ces aliments soient cuisinés, cuits au four ou transformés.

4) Les confiseries tels que le chocolat, les bonbons, les chewing-gums, les desserts, les nappages, les sorbets, les glaçages, et autres garnitures ;

5) Les boissons alcoolisées ou non, y compris les sodas et autres boissons non alcoolisées, jus de fruits, compléments diététiques, substituts de repas sous forme de breuvage comme ceux vendus sous la marque Boost™ and Ensure™ et ;

6) Les produits divers comme les oeufs, les aliments transformés comme les soupes, les sauces toute prête pour pâtes, des plats préparés et autre produits du même genre.

La composition de la présente invention peut être incorporée directement et sans autre modification dans la nourriture, les nutraceutiques, les produits diététiques notamment hyperprotéinés ou les breuvages et ce grâce à des techniques comme le mixage, l'infusion, l'injection, le mélange, l'absorption, le pétrissage et la pulvérisation.

Les modes d'administration, les posologies et les formes galéniques optimales des composés et compositions selon l'invention peuvent être déterminés selon les critères généralement pris en compte dans l'établissement d'un traitement pharmaceutique, en particulier dermatologique, ou vétérinaire adapté à un patient ou à un animal comme par exemple l'âge ou le poids corporel du patient ou de l'animal, la gravité de son état général, la tolérance au traitement, les effets secondaires constatés, le type de peau. En fonction du type d'administration souhaitée, la composition et/ou les composés actifs selon l'invention peuvent en outre comprendre au moins un excipient pharmaceutiquement acceptable, notamment dermatologiquement acceptable. Selon la première variante, on utilise un excipient adapté pour une administration par voie topique externe. La composition selon la présente invention peut en outre comprendre au moins un adjuvant pharmaceutiquement connu de l'homme du métier, choisi parmi les épaississants, les conservateurs, les parfums, les colorants, des filtres chimiques ou minéraux, les agents hydratants, les eaux thermales, etc.

La composition comprenant un extrait de fruit de *Schizandra sphenanthera* ayant les spécifications indiquées est particulièrement destinée à une utilisation cosmétique, dermatologique ou nutraceutique.

Dans le cadre d'une utilisation cosmétique ou dermatologique, la composition sera avantageusement formulée sous la forme d'une préparation adaptée à une administration topique. La composition comprenant un extrait peptidique et osidique est particulièrement destinée à une utilisation cosmétique ou dermatologique.

Dans le cadre d'une utilisation nutraceutique, à visée nutritive ou cosmétique (« cosmet-food), la composition sera avantageusement formulée sous la forme d'une préparation adaptée à une administration orale. Elle pourra ne pas comprendre d'excipient et être constituée, en intégralité, de l'huile de *Schizandra sphenanthera* raffinée.

L'invention a également pour objet l'utilisation d'un extrait peptidique et osidique de fruit de *Schizandra sphenanthera* pour la fabrication d'une composition cosmétique ou dermatologique ou alimentaire.

Dans un premier aspect, l'invention a pour objet l'utilisation d'un extrait peptidique et osidique de fruit de *Schizandra sphenanthera*, pour la prévention et le traitement des réactions ou pathologies allergiques, inflammatoires, irritatives, ou des troubles de la barrière ou de l'homéostasie de la peau et/ou des muqueuses et/ou des phanères immatures, normales ou matures/âgées dans une composition cosmétique.

Dans un deuxième aspect, l'invention a pour objet l'utilisation d'un extrait peptididique et osidique de fruit de *Schizandra sphenanthera*, , pour la prévention et le traitement des réactions ou pathologies allergiques, inflammatoires, irritatives, ou des troubles de la barrière ou de l'homéostasie de la peau et/ou des muqueuses et/ou des phanères immatures, normales ou matures/âgées dans une composition alimentaire telle qu'un aliment fonctionnel.

Un aliment fonctionnel est un aliment conventionnel, ou qui en a l'apparence, qui fait partie de l'alimentation normale, et qui a pour caractéristique de procurer des effets physiologiques bénéfiques dépassant ses fonctions nutritionnelles habituelles ou de réduire le risque de maladies chroniques.

En particulier, l'aliment fonctionnel est destiné à la prévention et au traitement des réactions ou pathologies allergiques, inflammatoires, irritatives ou des troubles de la barrière ou de l'homéostasie :

- de la peau, telles que l'acné, la rosacée (érythrocouperose), les rougeurs cutanées, le psoriasis, les troubles vasculaires, la dermite du siège, la dermatite atopique, l'eczéma, la dermatite de contact, la dermatite irritative, la dermatite allergique, la dermite séborrhéique (croûte de lait), le psoriasis, la peau sensible, la peau réactive, la peau sèche (xérose), la peau déshydratée, la peau avec rougeur, l'érythème cutanée, la peau âgée et photo-âgée, la peau photo sensibilisée, la peau pigmentée (melasma, pigmentation post-inflammatoire...), la peau dépigmentée (vitiligo), la peau avec cellulite, la peau relâchée, la peau avec vergetures, les dartre, les gerçures, les piqûres, les crevasses en particulier des seins, les coups de soleil, les inflammations dus aux rayons de toutes sortes, les irritations par agents chimiques, physiques (contrainte de tension : femmes enceintes), bactériologiques, fongiques ou viraux, parasitaires (poux, gale, teigne, acariens, dermatophytes), radiologiques ou par déficit de l'immunité innée (peptides antimicrobiens) ou acquise (cellulaire,humorales,cytokines), et/ou

- des muqueuses telles que les gencives et parodontes pouvant présenter des gingivites (gencives sensibles du nouveaux nés, problèmes d'hygiène, dues au tabagisme ou autres), des parodontopathies, ou des muqueuses génitales pouvant présenter des irritations des sphères génitales males ou femelles externes ou internes et/ou
- des phanères telles que les ongles (ongles cassants, fragiles ...) et les cheveux (alopécie, pellicules, hirsutismes, dermites séborrhéiques, folliculites) immatures, normales ou matures, présentant en particulier des troubles du cuir chevelu tels que les alopécies (ou pelade) androgénétique, aiguë, localisée, cicatricielle, congénitale, occipitale du nourisson, aerata, due à la chimiothérapie/radiothérapie ou encore l'effluvium télogène, l'effluvium anagène, la dystrophie pilaire, la trichotillomanie, la teigne ou les pellicules grasses ou sèches.

L'invention est également relative à un aliment ou une composition nutraceutique comprenant un extrait peptidique et osidique de fruit de *Schizandra sphenanthera*..

Dans un troisième aspect, l'invention a pour objet l'utilisation thérapeutique d'un extrait peptidique ou osidique de fruit de *Schizandra sphenanthera* pour la prévention et le traitement des réactions ou pathologies allergiques, inflammatoires, irritatives, ou des troubles de la barrière ou de l'homéostasie de la peau et/ou des muqueuses et/ou des phanères immatures, normales ou matures/âgées.

Selon un aspect préféré, l'invention a pour objet l'utilisation d'un extrait peptidique et osidique de fruit de *Schizandra sphenanthera* pour le traitement et la prévention des rougeurs cutanées, de l'érythro-couperose et de la rosacée dans une composition cosmétique.

Selon un autre aspect préféré, l'invention a pour objet l'utilisation d'un extrait peptidique et osidique de fruit de *Schizandra sphenanthera* pour le traitement et la prévention des rougeurs cutanées, de l'érythro-couperose et de la rosacée dans une composition alimentaire.

Selon un troisième aspect préféré, l'invention a pour objet l'utilisation thérapeutique d'un extrait peptidique et osidique de fruit de *Schizandra sphenanthera* pour le traitement et la prévention des rougeurs cutanées, de l'érythro-couperose et/ou de la rosacée.

## EXEMPLES

### <u>Exemple 1</u> : <u>Compositions pour application par voie topique</u>

Les inventeurs présentent ci-dessous plusieurs compositions pour application par voie topique. L'extrait peptidique et osidique de *Schizandra sphenanthera* peut être incorporé à divers produits cosmétiques tels que des eaux nettoyantes, des émulsions huile dans eau, des émulsions eau dans huile, des huiles, des laits, des lotions, des shampooings, des produits moussants et sprays, dont les compositions sont présentées ci-dessous.

**EAU NETTOYANTE HYDRATANTE**

| Nom commercial | % |
|---|---|
| EAU PURIFIÉE | QSP 100 % |
| BIOSACCHARID GUM | De 1 à 5 % |
| BUTYLENE GLYCOL | De 1 à 5 % |
| ACIDE HYALURONIQUE | De 0 à 5 % |
| **EXTRAIT PEPTIDIQUE ET OSIDIQUE DE SCHIZANDRA** | De 0,01 à 5 % |
| CONSERVATEURS | De 0 à 1 % |
| ACIDE CITRIQUE MONOHYDRATE | De 0 à 1 % |
| TROMETHAMINE | De 0 à 1 % |

**CREME LAVANTE PURIFIANTE**

| Matière première / Nom commercial | % |
|---|---|
| EAU PURIFIEE | QSP 100 % |
| ARLATONE | De 10 à 30 % |
| COCOGLUCOSIDE | De 5 à 20 % |

(suite)

| Matière première / Nom commercial | % |
|---|---|
| HYDROXYPROPYL GUAR | De 1 à 5 % |
| CAPRYLOYL GLYCINE | De 0 à 2 % |
| CONSERVATEURS | De 0 à 2 % |
| PARFUM | De 0 à 1 % |
| ACIDE CITRIQUE | De 0 à 1 % |
| ZINC PCA | De 0 à 1 % |
| **EXTRAIT PEPTIDIQUE ET OSIDIQUE DE SCHIZANDRA** | De 0,01 à 5 % |

**EMULSION ANTI-ACNEIQUE**

| Matière première / Nom commercial | % |
|---|---|
| PEG 40 STEARATE | De 1 à 5 % |
| PEG 5 GLYCERYL STEAR | De 1 à 5 % |
| CERESINE WAX | De 1 à 5 % |
| MONOSTEARATE DE GLYCEROL | De 1 à 5 % |
| STEARATE DE SORBITAN | De 0 à 2 % |
| ALCOOL CETYLIQUE | De 0 à 2 % |
| ALCOOL DI-MALATE | De 5 à 20 % |
| VITAMINE E | De 0 à 1 % |
| VITAMINE B3 | De 0 à 5 % |
| ACIDE LINOLEIQUE | De 0 à 1 % |
| **EXTRAIT PEPTIDIQUE ET OSIDIQUE DE SCHIZANDRA** | De 0,01 à 5 % |
| BUTYLENE GLYCOL | De 1 à 5 % |
| PIROCTOLAMINE | De 0 à 1 % |
| CONSERVATEURS | De 0 à 1 % |
| GLYCEROL | De 1 à 10 % |
| GOMME XANTHANE | De 0 à 1 % |
| ZINC PCA | De 0 à 2 % |
| AMIDON DE RIZ | De 1 à 5 % |
| NYLON 6 | De 0 à 2 % |
| POLYACRYLAMIDE GEL | De 1 à 5 % |
| VITAMINE B6 | De 0 à 1 % |
| PARFUM | De 0 à 1 % |
| EAU PURIFIÉE | QSP 100 % |

**SOIN GOMMANT**

| Matière première / Nom commercial | % |
|---|---|
| ARLATONE DUO | De 5 à 20 % |

(suite)

| Matière première / Nom commercial | % |
|---|---|
| AGENT GOMMANT | De 1 à 10 % |
| SCLEROTIUM GUM | De 1 à 10 % |
| CONSERVATEURS | De 0 à 1 % |
| CAPRYLOYL GLYCINE | De 0 à 1 % |
| SOUDE | De 0 à 1 % |
| **EXTRAIT PEPTIDIQUE ET OSIDIQUE DE SCHIZANDRA** | **De 0,01 à 5 %** |
| SEQUESTRANT | De 0 à 1 % |
| ACIDE CITRIQUE | De 0 à 1 % |
| EAU PURIFIÉE | QSP 100 % |
| PARFUM | De 0 à 1 % |

**CREME SOLAIRE SPF 50+**

| Matière première / Nom commercial | % |
|---|---|
| EAU PURIFIÉE B4 | QSP 100% |
| OXYDE DE TITANE | De 10 à 20% |
| CYCLOPENTASILOXANE | De 5 à 15% |
| OCTYL PALMITATE | De 5 à 15% |
| C12-C15 ALKYL BENZOATE | De 5 à 10% |
| DECYL PENTANOATE | De 5 à 10% |
| OXYDE DE ZINC | De 5 à 10% |
| GLYCEROL | De 1 à 5% |
| PEG-45/DODECYL GLYCOL COPOLYMERE | De 1 à 5% |
| **EXTRAIT PEPTIDIQUE ET OSIDIQUE DE SCHIZANDRA** | **De 0,01 à 5 %** |
| CHLORURE DE SODIUM | De 1 à 5% |
| DEXTRIN PALMITATE | De 1 à 2% |
| VITAMINE E | De 0 à 2 % |
| CONSERVATEURS | De 0 à 2 % |
| HYDROXYPROPYL GUAR | De 0 à 1 % |
| ALOE VERA | De 0 à 1 % |
| LESSIVE SOUDE | De 0 à 1 % |
| EDTA 2 Na | De 0 à 1 % |
| GLUCONATE ZINC | De 0 à 1 % |

**EMULSION ANTI-ROUGEURS**

| Matière première / Nom commercial | % |
|---|---|
| PEG 40 STEARATE | De 1 à 5 % |
| PEG 5 GLYCERYL STEAR | De 1 à 5 % |

(suite)

| Matière première / Nom commercial | % |
|---|---|
| CERESINE WAX | De 1 à 5 % |
| MONOSTEARATE DE GLYCEROL | De 1 à 5 % |
| STEARATE DE SORBITAN | De 0 à 2 % |
| ALCOOL CETYLIQUE | De 0 à 2 % |
| ALCOOL DI-MALATE | De 5 à 20 % |
| SOPHORA JAPONICA | De 0 à 5 % |
| VITAMINE | De 0 à 1 % |
| **EXTRAIT PEPTIDIQUE ET OSIDIQUE DE SCHIZANDRA** | De 0,01 à 5 % |
| BUTYLENE GLYCOL | De 1 à 5 % |
| PIROCTOLAMINE | De 0 à 1 % |
| CONSERVATEURS | De 0 à 1 % |
| GLYCEROL | De 1 à 10 % |
| GOMME XANTHANE | De 0 à 1 % |
| ZINC PCA | De 0 à 2% |
| AMIDON DE RIZ | De 1 à 5 % |
| NYLON 6 | De 0 à 2% |
| POLYACRYLAMIDE GEL | De 1 à 5 % |
| VITAMINE B6 | De 0 à 1 % |
| PARFUM | De 0 à 1 % |
| EAU PURIFIÉE | QSP 100 % |

**EMULSION ANTI-AGE ANTI-ROUGEURS**

| Matière première / Nom commercial | % |
|---|---|
| ISOPARAFFINE LIQUIDE | De 5 à 20 % |
| STEARATE D’ISOCETYLE | De 5 à 20 % |
| HYDROXYSTEARATE AL - MG | De 5 à 20 % |
| ABIL WE 09 | De 1 à 5 % |
| GLYCEROL | De 1 à 5 % |
| HUILE VASELINE | De 1 à 5 % |
| ZINC OXYDE MICRONISE | De 1 à 5 % |
| BUTYLENE GLYCOL | De 1 à 5 % |
| RETINOL | De 0 à 1% |
| VITAMINE C | De 0 à 5% |
| **EXTRAIT PEPTIDIQUE ET OSIDIQUE DE SCHIZANDRA** | De 0,01 à 5 % |
| ISONONYL ISONONANOAT | De 1 à 5 % |
| CIRE D’ABEILLE | De 1 à 5 % |
| TARTRATE DE SODIUM | De 1 à 5 % |

(suite)

| Matière première / Nom commercial | % |
|---|---|
| CHLORURE DE SODIUM | De 0 à 5 % |
| GLYCINE | De 1 à 5 % |
| CONSERVATEURS | De 0 à 1 % |
| CHOLESTEROL | De 0 à 1 % |
| PHYTOSPHINGOSINE | De 0 à 1 % |
| ACIDE TARTRIQUE | De 0 à 1 % |
| EAU PURIFIÉE | QSP 100 % |

**Exemple 2 : Compositions pour administration par voie orale**

Les peptides de Schizandra sont intégrés à des compositions orales, dans des compositions permettant l'administration de 50 mg à 200 mg d'extrait de Schizandra par jour.

**2.1 Composition anti-vergetures sous forme de capsules molles**

| | |
|---|---|
| - **EXTRAIT PEPTIDIQUE ET OSIDIQUE DE SCHIZANDRA** | 30 mg |
| - Huile d'Awara | 60 mg |
| - Huile de colza riche en insaponifiable | 300 mg |
| - Vitamine du groupe B (B1, B2, B3, B5, B6, B9, B12), | QSP 100% des AJR |
| - Tocotriénols | QSP 50 % AJR |
| - Vitamine E | |
| - Cire d'abeille | |
| - Lécithine de soja | |
| - Gélatine alimentaire | |
| - Glycérine | QSP 1 capsule molle |

Cette composition est administrée de 4 à 6 capsules de 500 mg par jour.

**2.2/ Exemples en stick poudre amincissant**

| | |
|---|---|
| - **EXTRAIT PEPTIDIQUE ET OSIDIQUE DE SCHIZANDRA** | 100 mg |
| - Extrait de thé riche en polyphénols | 100 mg |
| - Extrait de raisin riche en OPC | 50 mg |
| - Bétaglucanes d'origine végétale | 100 mg |
| - Gomme xanthane | 1 mg |
| - ascorbate de sodium | 0,3 mg |
| - maltodextrine | QSP 5 g |

Cette composition est administrée 2 fois par jour.

**Exemple 3 - Activité d'un extrait peptidique et osidique de *Schizandra sphenanthera* dans la phase inflammatoire de la rosacée**

Dans les exemples 3 à 5, l'expression « Peptides de Schizandra » désigne l'extrait peptidique et osidique de *Schizandra sphenanthera* comprenant un certain pourcentage de peptides.

Afin d'évaluer l'activité potentielle des Peptides de Schizandra dans la phase inflammatoire de la rosacée, nous avons étudié l'effet de ce complexe d'actifs sur l'expression génique de la kallikréine 5 (KLK5) et de la cathélicidine LL37 induites par un analogue de la vitamine D (calcitriol) dans des kératinocytes.

**Matériel et méthodes**

Des kératinocytes épidermiques humains normaux (NHEK) ont été cultivés en milieu de culture supplémenté en Ca++ afin de les amener à un état de différenciation.
Les kératinocytes ont été traités pendant 24 heures par les Peptides de Schizandra à 0,05% (p/v) en présence ou non de Calcitriol à 10-7M (analogue de la vitamine D).
A la fin du traitement, les surnageants de culture ont été éliminés et les ARN totaux extraits à l'aide du kit d'extraction RNeasy MiniKit [Qiagen]. Les ARN totaux ont ensuite été dosés en minichips à l'aide du système Experion™ et du kit Experion RNA StdSens [Biorad] puis réverse-transcrits en cDNA à l'aide du kit iScript cDNA Synthesis [Biorad].
Les cDNA néo-synthétisés relatifs aux gènes d'intérêt (KLK5 et LL37) ou aux gènes de référence ont été amplifiés sélectivement par PCR en temps réel sur iQ5 [Biorad] par la technologie SybrGreen [kit iQ SybrGreen, Biorad].
La méthode de RT-PCR en temps réel permet la quantification relative du niveau d'expression d'un gène d'intérêt par rapport à celui d'un gène de référence en réponse à un traitement donné.
L'analyse quantitative des résultats est basée sur le recueil des cycles seuils (ou Ct pour threshold cycle).
Le cycle seuil correspond au point où le signal d'émission de fluorescence est statistiquement et significativement plus élevé que le bruit de fond. Le cycle seuil est directement corrélé au nombre de copies initiales de l'ADN cible.
Pour chaque échantillon, le niveau d'expression du gène d'intérêt a été normalisé par le niveau d'expression du gène de référence le plus stable. Le gène de référence le plus stable a été déterminé à l'aide de la macro GeNorm, il s'agit du gène GAPDH.
On calcule ainsi le ΔCt :

$$\Delta Ct = Ct \text{ gène cible} - Ct \text{ gène de référence}$$

Dans une deuxième étape, la variation, en fonction du traitement, du nombre de copies du gène d'intérêt a été déterminée. On calcule ainsi le ΔΔCt :

$$\Delta\Delta Ct = \Delta Ct \text{ contrôle} - \Delta Ct \text{ traité}$$

Enfin, la quantité relative (QR) est calculée : QR = (1+E)ΔΔCt.
On considère que E (l'efficacité) est égale à 1, on a donc :

$$QR = 2\Delta\Delta Ct$$

La significativité des résultats a été vérifiée sur les valeurs de ΔCt, par une analyse de variance à un facteur suivie d'un test de Tuckey (logiciel GraphPad Prism version 5.02, GraphPad Software, San Diego California USA).

**Résultats**

**Expression génique de KLK5 :**

Le calcitriol (analogue de la vitamine D) a fortement et significativement stimulé l'expression génique de la kallikréine 5 par les kératinocytes (+149%, p<0,01).
Les Peptides de Schizandra ont significativement inhibé l'expression de la KLK5 induite par le calcitriol (54% d'inhibition, p<0,01).

| | Moyenne ΔCt (CtKLK5 - CtGAPDH) | Ecart type | ΔΔCt | Expression de KLK5 (QR) |
|---|---|---|---|---|
| Cellules contrôles | 3,78 | 0,99 | 0,00 | 1 |
| Témoin stimulé (Calcitriol) | 2,46 | 1,35 | 1,32 | 2,49 $$ |

(suite)

| | Moyenne ΔCt (CtKLK5 - CtGAPDH) | Ecart type | ΔΔCt | Expression de KLK5 (QR) |
|---|---|---|---|---|
| Peptides Schizandra 0,05% + Calcitriol | 3,57 | 0,63 | 0,21 | 1,16 ** |
| $$ p<0,01 vs cellules contrôles<br>** p<0,01 vs témoin stimulé | | | | |

**Expression génique de LL37 :**

Le calcitriol a fortement et significativement stimulé l'expression génique de la LL37 par les kératinocytes (+795%, p<0,001).

Les Peptides de Schizandra ont significativement inhibé l'expression de la LL37 induite par le calcitriol (58% d'inhibition, p<0,01).

| | Moyenne ΔCt (CtLL37 - CtGAPDH) | Ecart type | ΔΔCt | Expression de LL37 (QR) |
|---|---|---|---|---|
| Cellules contrôles | 14,17 | 0,69 | 0,00 | 1 |
| Témoin stimulé (Calcitriol) | 11,01 | 0,28 | 3,16 | 8,95 $$$ |
| Peptides Schizandra 0,05% + Calcitriol | 12,27 | 1,02 | 1,90 | 3,74 ** |
| $$$ p<0,001 vs cellules contrôles<br>** p<0,01 vs témoin stimulé | | | | |

**Conclusion**

Dans ces conditions expérimentales, les Peptides de Schizandra ont permis de moduler l'expression d'un marqueur augmenté dans la rosacée : la cathélicidine LL37 ainsi que de l'enzyme responsable de sa maturation : la kallikréine 5.

Ainsi, les Peptides de Schizandra pourraient contribuer à moduler l'induction de la réponse inflammatoire dans la rosacée.

**Exemple 4 : Activité d'un extrait peptidique et osidique de Schizandra Sphenanthera sur d'autres paramètres inflammatoires :**

**Matériel et Méthodes :**

Des kératinocytes humains normaux ont été pré-incubés pendant 24 heures avec les Peptides de Schizandra aux concentrations de 0,005% et 0,05% ou avec la dexaméthasone à 10-7 M (molécule de référence anti-inflammatoire).

L'inflammation a ensuite été induite par traitement au PMA à 10 μg/ml pendant 24 heures, toujours en présence des Peptides de Schizandra ou de dexaméthasone.

A la fin de l'incubation, les quantités d'interleukine-1β (IL1β), d'interleukine-8 (IL8) et d'interleukine-6 (IL6) présentes dans les surnageants de culture ont été mesurées par ELISA.

Parallèlement, le nombre de cellules biologiquement actives est déterminé par un test au Rouge Neutre. La quantité de cytokine dosée pour chaque condition est ramenée au nombre de cellules vivantes par division par la valeur de DO540 obtenue à l'issue du test au rouge neutre.

La significativité des résultats a été vérifiée par une analyse de variance à un facteur suivie d'un test de Tuckey (logiciel GraphPad Prism version 5.02, GraphPad Software, San Diego California USA).

**Résultats**

**Sécrétion d'IL1β:**

Le traitement par le PMA a nettement stimulé la libération d'IL1β par les kératinocytes, la dexaméthasone a nettement inhibé cette libération (-65%), ces résultats valident l'essai.

Les Peptides de Schizandra ont significativement inhibé la sécrétion d'IL1β induite par le PMA sur des kératinocytes aux deux concentrations testées : 64% (p<0,001) à 0,005% et 56% (p<0,001) à 0,05% d'inhibition.

| | IL1β (DOELISA / Neutre) | DORouge |
|---|---|---|
| Cellules contrôles | 0,049 ± 0,008 | |
| Témoin stimulé (PMA) | 0,106 ± 0,008 | $$$ |
| Dexaméthasone 10-7M | 0,037 ± 0,011 | -65% *** |
| Peptides de Schizandra 0,005% | 0,039 ± 0,001 | -64% *** |
| Peptides de Schizandra 0,05% | 0,047 ± 0,009 | -56% *** |
| $$$ p<0,001 vs cellules contrôles<br>*** p<0,001 vs témoin stimulé | | |

**Sécrétion d'IL8 :**

Le traitement par le PMA a nettement stimulé la libération d'IL8 par les kératinocytes, la dexaméthasone a nettement inhibé cette libération (-53%), ces résultats valident l'essai.

Les Peptides de Schizandra ont significativement inhibé le relargage d'IL8 induit par le PMA aux deux concentrations testées : 58% (p<0,001) à 0,005% et 37% (p<0,001) à 0,05% d'inhibition.

| | IL8 (pg/ml / Neutre) | DORouge |
|---|---|---|
| Cellules contrôles | 0,515 ± 0,080 | |
| Témoin stimulé (PMA) | 2,056 ± 0,166 | $$$ |
| Dexaméthasone 10-7M | 0,973 ± 0,213 | -53% *** |
| Peptides de Schizandra 0,005% | 0,871 ± 0,032 | -58% *** |
| Peptides de Schizandra 0,05% | 1,285 ± 0,159 | -37% *** |
| $$$ p<0,001 vs cellules contrôles<br>*** p<0,001 vs témoin stimulé | | |

**Sécrétion d'IL6 :**

Le traitement par le PMA a significativement induit la sécrétion d'IL6 par les kératinocytes, la dexaméthasone a inhibé ce relargage, ces résultats valident l'essai.

Les Peptides de Schizandra ont fortement et significativement inhibé le relargage d'IL6 induit par le PMA aux deux concentrations testées : -70% (p<0,001) à 0,005% et -74% (p<0,001) à 0,05%.

| | IL6 (DOELISA / Neutre) | DORouge |
|---|---|---|
| Cellules contrôles | 0,069 ± 0,003 | |
| Témoin stimulé (PMA) | 0,098 ± 0,002 | $$ |
| Dexaméthasone 10-7M | 0,043 ± 0,012 | -56% *** |
| Peptides de Schizandra 0,005% | 0,029 ± 0,003 | -70% *** |

(suite)

| | IL6 (DOELISA / Neutre) | DORouge |
|---|---|---|
| Peptides de Schizandra 0,05% | 0,025 ± 0,004 | -74% *** |
| $$ p<0,01 vs cellules contrôles<br>*** p<0,001 vs témoin stimulé | | |

Conclusion

Dans ces conditions expérimentales, les Peptides de Schizandra ont présenté un effet modulateur de l'inflammation au niveau d'une cytokine primaire (IL1β) et de deux cytokines secondaires (IL8 et IL6).

**Exemple 5 : Activité d'un extrait peptidique et osidique de Schizandra Sphenanthera sur les paramètres vasculaires**

**5.1. Limitation de la prolifération des cellules endothéliales**

Matériel et méthodes

Des cellules endothéliales micro-vasculaires dermiques humaines normales ont été incubées pendant 24 ou 48 heures en absence (contrôle) ou en présence des Peptides de Schizandra aux concentrations de 0,05% et 0,1% et en présence et en absence du VEGF à 10 ng/ml (Vascular Endothelial Growth Factor), activateur de référence.
A la fin de l'incubation, la viabilité des cellules a été évaluée par une méthode spectrophotométrique de dosage de l'activité des phosphatases intracellulaires.
La significativité statistique a été évaluée par une analyse de variance à un facteur suivie d'un test de Holm-Sidak.

Résultats

Prolifération des cellules endothéliales en absence de VEGF :

En conditions basales (sans VEGF), la croissance des cellules endothéliales a été significativement diminuée après 24 heures de traitement par les Peptides de Schizandra aux deux concentrations testées :

| | | |
|---|---|---|
| 24 heures | Peptides de Schizandra 0,05% | -89,1% (p<0,05) |
| | Peptides de Schizandra 0,1% | -112,7% (p<0,05) |

Prolifération des cellules endothéliales en présence de VEGF :

Le VEGF a significativement stimulé la croissance des cellules endothéliales par rapport aux cellules contrôles : +41,4% (p<0,01) après 24 heures d'incubation et +96,3% (p<0,01) après 48 heures d'incubation. Ce résultat valide l'essai.
Les Peptides de Schizandra, aux deux concentrations testées, ont significativement inhibé la prolifération des cellules endothéliales induite par le VEGF :

| | | |
|---|---|---|
| 24 heures | Peptides de Schizandra 0,05% | -51,8% (p<0,05) |
| | Peptides de Schizandra 0,1% | -81,8% (p<0,05) |
| 48 heures | Peptides de Schizandra 0,05% | -21,6% (p<0,05) |
| | Peptides de Schizandra 0,1% | -27,1% (p<0,05) |

Conclusion

Les Peptides de Schizandra en limitant la prolifération des cellules endothéliales pourraient limiter la phase précoce de l'angiogénèse.

### 5.2. Effet sur l'expression de marqueurs de l'angiogenèse par des keratinocytes

Afin de comprendre l'effet anti-angiogénique des Peptides de Schizandra, l'expression génique ou protéique par des kératinocytes de certains marqueurs impliqués dans l'angiogénèse a été étudiée, en particulier l'expression des facteurs de croissance pro-angiogéniques VEGF et FGF2 et d'un marqueur anti-angiogénique (Thrombospondine-1).

### A. Expression génique de marqueurs pro-angiogéniques (VEGF, FGF2)

**Matériel et méthodes**

Des kératinocytes épidermiques humains normaux (NHEK) ont été incubés pendant 48h en présence des Peptides de Schizandra à 0,05% et 0,1% (p/v).

A la fin du traitement, les surnageants de culture ont été éliminés et les ARN totaux extraits à l'aide du kit d'extraction RNeasy MiniKit [Qiagen]. Les ARN totaux ont ensuite été dosés en minichips à l'aide du système Experion™ et du kit Experion RNA StdSens [Biorad] puis réverse-transcrits en cDNA à l'aide du kit iScript cDNA Synthesis [Biorad].

Les cDNA néo-synthétisés relatifs aux gènes d'intérêt (VEGF, FGF2, HIF1$\alpha$, THBS1) ou aux gènes de référence ont été amplifiés sélectivement par PCR en temps réel sur iQ5 [Biorad] par la technologie SybrGreen [kit iQ SybrGreen, Biorad].

La méthode de RT-PCR en temps réel permet la quantification relative du niveau d'expression d'un gène d'intérêt par rapport à celui d'un gène de référence en réponse à un traitement donné.

L'analyse quantitative des résultats est basée sur le recueil des cycles seuils (ou Ct pour threshold cycle).

Le cycle seuil correspond au point où le signal d'émission de fluorescence est statistiquement et significativement plus élevé que le bruit de fond. Le cycle seuil est directement corrélé au nombre de copies initiales de l'ADN cible.

Pour chaque échantillon, le niveau d'expression du gène d'intérêt a été normalisé par le niveau d'expression du gène de référence le plus stable. Le gène de référence le plus stable a été déterminé à l'aide de la macro GeNorm, il s'agit du gène YWHAZ.

On calcule ainsi le $\Delta$Ct :

$$\Delta Ct = Ct \text{ gène cible} - Ct \text{ gène de référence}$$

Dans une deuxième étape, la variation, en fonction du traitement, du nombre de copies du gène d'intérêt a été déterminée. On calcule ainsi le $\Delta\Delta$Ct :

$$\Delta\Delta Ct = \Delta Ct \text{ contrôle} - \Delta Ct \text{ traité}$$

Enfin, la quantité relative (QR) est calculée : QR = (1+E)$\Delta\Delta$Ct.

On considère que E (l'efficacité) est égale à 1, on a donc :

$$QR = 2\Delta\Delta Ct$$

La significativité des résultats a été vérifiée sur les valeurs de $\Delta$Ct, par une analyse de variance à un facteur suivie d'un test de Dunnett (logiciel GraphPad Prism version 5.02, GraphPad Software, San Diego California USA).

**Résultats**

**Expression génique du VEGF (Vascular Endothelial Growth Factor) :**

Les Peptides de Schizandra ont très fortement et significativement inhibé l'expression génique du VEGF par des kératinocytes aux deux concentrations testées : 89% à 0,05% ($p<0,001$) et 93% à 0,1% ($p<0,001$) d'inhibition.

| | Moyenne $\Delta$CT (CTVEGF - CTYWHAZ) | Ecart type | $\Delta\Delta$CT | Expression du VEGF (QR) |
|---|---|---|---|---|
| Cellules contrôles | 8,41 | 2,09 | 0,00 | 1 |

(suite)

| | Moyenne ΔCT (CTVEGF - CTYWHAZ) | Ecart type | ΔΔCT | Expression du VEGF (QR) |
|---|---|---|---|---|
| Peptides de Schizandra 0,05% | 11,63 | 2,91 | -3,23 | 0,107*** |
| Peptides de Schizandra 0,1% | 12,21 | 2,60 | -3,80 | 0,072*** |
| *** p<0,001 vs cellules contrôles | | | | |

**Expression génique du FGF2 (basic Fibroblast Growth Factor) :**

Les Peptides de Schizandra ont très fortement et significativement inhibé l'expression du FGF2 par des kératinocytes aux deux concentrations testées : 93% à 0,05% (p<0,001) et 95% à 0,1% (p<0,001) d'inhibition.

| | Moyenne ΔCT (CTFGF2 - CTYWHAZ) | Ecart type | ΔΔCT | Expression génique du FGF2 (QR) |
|---|---|---|---|---|
| Cellules contrôles | 4,57 | 0,46 | 0,00 | 1 |
| Peptides de Schizandra 0,05% | 8,31 | 0,83 | -3,75 | 0,075 *** |
| Peptides de Schizandra 0,1% | 8,86 | 1,00 | -4,30 | 0,051 *** |
| *** p<0,001 vs cellules contrôles | | | | |

**Expression génique de la thrombospondine-1** :

Les Peptides de Schizandra ont significativement stimulé l'expression de la thrombospondine-1, facteur anti-angiogénique, par des kératinocytes aux deux concentrations testées : +118% (p<0,01) à 0,05% et +60% (p<0,05) à 0,1%.

| | Moyenne ΔCT (CTTHBS-1 - CTYWHAZ) | Ecart type | ΔΔCT | Expression de la THBS1 (QR) |
|---|---|---|---|---|
| Cellules contrôles | -0,85 | 0,19 | 0,00 | 1 |
| Peptides de Schizandra 0,05% | -1,98 | 0,50 | 1,13 | 2,181 ** |
| Peptides de Schizandra 0,1% | -1,54 | 0,51 | 0,68 | 1,605 * |
| * p<0,05 ; ** p<0,01 vs cellules contrôles | | | | |

**B. Expression protéique d'un marqueur de l'angiogénèse (VEGF)**

**Matériel et méthodes**

Des kératinocytes humains normaux ont été pré-incubés pendant 24 heures avec les Peptides de Schizandra aux concentrations de 0,005% ; 0,05% et 0,1% ou avec la dexaméthasone à 10-7 M (molécule de référence anti-inflammatoire).

L'inflammation a ensuite été induite par traitement au PMA à 10 μg/ml pendant 24 heures, toujours en présence des Peptides de Schizandra ou de dexaméthasone.

A la fin de l'incubation, la quantité de VEGF présentes dans les surnageants de culture a été mesurée par ELISA.

Parallèlement, le nombre de cellules biologiquement actives est déterminé par un test au Rouge Neutre. La quantité de cytokine dosée pour chaque condition est ramenée au nombre de cellules vivantes par division par la valeur

de DO540 obtenue à l'issue du test au rouge neutre.

La significativité des résultats a été vérifiée par une analyse de variance à un facteur suivie d'un test de Tuckey (logiciel GraphPad Prism version 5.02, GraphPad Software, San Diego California USA).

## Résultats

Le traitement par le PMA a significativement induit la sécrétion du VEGF par les kératinocytes, la dexaméthasone a inhibé ce relargage (-46%), ces résultats valident l'essai. Les Peptides de Schizandra ont fortement et significativement inhibé le relargage de VEGF induit par le PMA aux trois concentrations testées : respectivement 63%, 72% et 69% d'inhibition (p<0,001).

| | VEGF (pg/ml / Neutre) | DORouge |
|---|---|---|
| Cellules contrôles | 2521,921 ± 317,263 | |
| Témoin stimulé (PMA) | 3333,861 ± 111,766 | $ |
| Dexaméthasone 10-7M | 1789,338 ± 489,677 | -46% *** |
| Peptides de Schizandra 0,005% | 1230,425 ± 35,305 | -63% *** |
| Peptides de Schizandra 0,05% | 928,914 ± 106,597 | -72% *** |
| Peptides de Schizandra 0,1% | 1027,362 ± 34,258 | -69% *** |
| $ p<0,05 vs cellules contrôles<br>*** p<0,001 vs témoin stimulé | | |

## C. Conclusion

Dans les conditions expérimentales présentées ci-dessus, nous avons pu montrer que les Peptides de Schizandra pouvaient moduler au niveau des kératinocytes l'expression de facteurs impliqués dans l'induction de l'angiogénèse, en faveur d'un effet anti-angiogénique.

En effet, les Peptides de Schizandra ont pu diminuer l'expression des marqueurs pro-angiogéniques (FGF2, VEGF) et augmenter l'expression d'un marqueur anti-angiogénique (THBS1).

Ainsi en défavorisant l'angiogénèse, phénomène précoce de la néovascularisation, les Peptides de Schizandra pourraient limiter l'activation des cellules endothéliales et la synthèse de nouveaux vaisseaux sanguins.

### 5.3. Effet sur la vasodilatation : expression de l'endothéline-1

L'endothéline est un neuropeptide qui a une activité vasoconstrictrice puissante, elle est sécrétée par les cellules endothéliales, épithéliales (kératinocytes), fibroblastes, adipocytes, et les macrophages. L'effet des Peptides de Schizandra sur la production d'endothéline par des cellules endothéliales a été étudié.

## Matériel et méthodes

Des cellules endothéliales micro-vasculaires dermiques humaines normales adultes cultivées en monocouche ont été incubées pendant 6 heures en absence (contrôle) ou en présence des Peptides de Schizandra à 0,05% et 0,1% ou d'insuline à 16,7nM (activateur de référence).

A la fin de la période d'incubation, l'endothéline relarguée a été quantifiée par ELISA. En parallèle les protéines contenues dans les lysats cellulaires ont été quantifiées par la méthode de Bradford.

La quantité d'endothéline dosée a donc été normalisée par la quantité de protéines totales, les résultats ont donc été exprimés sous forme du rapport ng d'endothéline / μg de protéines totales du tapis cellulaire.

La significativité statistique des résultats a été évaluée par une analyse de variance à un facteur suivie d'un test de Holm-Sidak.

## Résultats

Les Peptides de Schizandra ont significativement augmenté la production de l'endothéline par les cellules endothéliales aux deux concentrations testées : +67% (p<0,05) à 0,05% et +78% (p<0,05) à 0,1%.

| | Endothéline (ng/µg de protéines) | |
|---|---|---|
| Cellules contrôles | 1,33 ± 0,11 | |
| Insuline 16,7 nM | 1,72 ± 0,23 | +30% |
| Peptides de Schizandra 0,05% | 2,22 ± 0,27 | +67% * |
| Peptides de Schizandra 0,1% | 2,36 ± 0,15 | +78% * |
| * p<0,05 vs cellules contrôles | | |

**Conclusion**

Par un effet activateur de l'expression d'endothéline, les Peptides de Schizandra pourraient avoir un effet régulateur de la vasodilatation en faveur d'une diminution des rougeurs cutanées.

## Revendications

1. Composition cosmétique, dermatologique ou nutraceutique comprenant au moins un extrait peptidique et osidique de fruit de *Schizandra sphenanthera*, ne comprenant pas de lignanes.

2. Composition selon la revendication 1, **caractérisée en ce que** l'extrait peptidique et osidique est constitué de 10 à 50 % en poids de peptides et de 10 à 60 % en poids de sucres, les pourcentages étant exprimés par rapport au poids total dudit extrait peptidique.

3. Composition cosmétique, dermatologique ou nutraceutique selon l'une des revendications 1 à 2 comprenant au moins un autre composé actif en plus de l'extrait peptidique et osidique de fruit de *Schizandra sphenanthera.*

4. Composition cosmétique, dermatologique ou nutraceutique selon l'une des revendications 1 à 3 comprenant au moins deux extraits de fruit de *Schizandra sphenanthera*, le deuxième extrait étant choisi parmi une huile brute, une huile raffinée, un concentrat d'huile raffinée et un insaponifiable.

5. Procédé de préparation d'un extrait peptidique et osidique de fruit de *Schizandra sphenanthera* comprenant les étapes successives suivantes :

   - à partir de fruit de *Schizandra* extraction par $CO_2$ supercritique d'une huile brute et récupération d'un tourteau délipidé ;
   - dispersion en phase aqueuse dudit tourteau ;
   - traitement enzymatique dudit tourteau par un mélange enzymatique de cellulases, protéases, alpha-amylases, puis
   - centrifugation, ultrafiltration et diafiltration, nanofiltration et
   - récupération de l'extrait peptidique.

6. Composition comprenant un extrait peptidique et osidique de fruit de *Schizandra sphenanthera*, pour son utilisation dans la prévention et le traitement des réactions ou pathologies allergiques, inflammatoires, irritatives, ou des troubles de la barrière ou de l'homéostasie de la peau et/ou des muqueuses et/ou des phanères immatures, normales ou matures/âgées.

7. Extrait peptidique et osidique de fruit de *Schizandra sphenanthera* pour son utilisation dans le traitement et la prévention des rougeurs cutanées, par administration topique ou orale.

8. Composition thérapeutique comprenant un extrait peptidique et osidique de fruit de *Schizandra sphenanthera* pour son utilisation dans le traitement et la prévention de l'érythro-couperose et/ou de la rosacée.

## Patentansprüche

1. Kosmetische, dermatologische oder nutrazeutische Zusammensetzung, die mindestens ein peptidisches und osidisches *Schisandra-sphenanthera*-Fruchtextrakt umfasst, die keine Lignane umfasst.

2. Zusammensetzung nach Anspruch 1, **dadurch gekennzeichnet, dass** das peptidische und osidische Extrakt zu 10 bis 50 Gew.-% aus Peptiden und zu 10 bis 60 Gew.-% aus Zuckern besteht, wobei die Prozentsätze bezogen auf das Gesamtgewicht des peptidischen Extrakts ausgedrückt sind.

3. Kosmetische, dermatologische oder nutrazeutische Zusammensetzung nach einem der Ansprüche 1 bis 2, die mindestens einen anderen Wirkstoff zusätzlich zu dem peptidischen und osidischen *Schisandra-sphenanthera*-Fruchtextrakt umfasst.

4. Kosmetische, dermatologische oder nutrazeutische Zusammensetzung nach einem der Ansprüche 1 bis 3, die mindestens zwei *Schisandra-sphenanthera*-Fruchtextrakte umfasst, wobei der zweite Extrakt unter einem Rohöl, einem raffinierten Öl, einem Konzentrat aus raffiniertem Öl und einem unverseifbaren Stoff ausgewählt ist.

5. Verfahren zur Zubereitung eines peptidischen und osidischen *Schisandra-sphenanthera*-Fruchtextrakts, das die folgenden aufeinanderfolgenden Schritte umfasst:

   - ausgehend von der *Schisandra*-Frucht, Extraktion eines Rohöls durch überkritisches $CO_2$ und Rückgewinnung eines delipidierten Kuchens;
   - Dispersion des Kuchens in der wässrigen Phase;
   - enzymatische Behandlung des Kuchens durch eine enzymatische Mischung aus Cellulasen, Proteasen, Alpha-Amylasen, dann
   - Zentrifugierung, Ultrafiltration und Diafiltration, Nanofiltration und
   - Rückgewinnung des peptidischen Extrakts.

6. Zusammensetzung, die ein peptidisches und osidisches *Schisandra-sphenanthera*-Fruchtextrakt umfasst, für ihre Verwendung bei der Prävention und Behandlung von allergischen, inflammatorischen, irritativen Reaktionen oder Krankheiten oder Störungen der Hautbarriere oder -homöostase und/oder der Schleimhäute und/oder unreifen, normalen oder reifen/gealterten Anhangsgebiete der Haut.

7. Peptidisches und osidisches *Schisandra-sphenanthera*-Fruchtextrakt für seine Verwendung bei der Behandlung und der Prävention von Hautrötungen durch topische oder orale Verabreichung.

8. Therapeutische Zusammensetzung, die ein peptidisches und osidisches *Schisandra-sphenanthera*-Fruchtextrakt umfasst, für seine Verwendung bei der Behandlung und der Prävention von Erythro-Couperose und/oder Rosacea.

## Claims

1. Cosmetic, dermatological or nutraceutical composition comprising at least one *Schisandra sphenanthera* fruit peptide and sugar extract, not comprising lignans.

2. Composition according to claim 1, **characterized in that** the peptide and sugar extract consists of 10% to 50% by weight of peptides and 10% to 60% by weight of sugars, wherein the percentages are expressed in relation to the total weight of said peptide extract.

3. Cosmetic, dermatological or nutraceutical composition according to claim 1 or claim 2, comprising at least one other active compound in addition to the *Schisandra sphenanthera* fruit peptide and sugar extract.

4. Cosmetic, dermatological or nutraceutical composition according to one of claims 1 to 3, comprising at least two *Schisandra sphenanthera* fruit extracts, wherein the second extract is selected from a crude oil, a refined oil, a refined oil concentrate and an unsaponifiable.

5. Method for the preparation of a *Schisandra sphenanthera* fruit peptide and sugar extract, comprising the following successive steps:

- starting with *Schisandra* fruit, extraction by supercritical $CO_2$ of a crude oil and recovery of a defatted oil cake;
- aqueous phase dispersion of said oil cake;
- enzymatic treatment of said oil cake with an enzymatic mixture of cellulases, proteases and alpha-amylases, then
- centrifugation, ultrafiltration and diafiltration, nanofiltration and
- recovery of the peptide extract.

**6.** Composition comprising a *Schisandra sphenanthera* fruit peptide and sugar extract, for use in the prevention and treatment of allergic, inflammatory or irritative reactions or pathologies, or of disorders of the barrier or the homeostasis of the skin and/or the mucosa and/or of immature, normal or mature/aged epithelial appendages.

**7.** *Schisandra sphenanthera* fruit peptide and sugar extract for use in the treatment and prevention of cutaneous redness, by topical or oral administration.

**8.** Therapeutic composition comprising a *Schisandra sphenanthera* fruit peptide and sugar extract for use in the treatment and prevention of erythro-couperose and/or rosacea.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 2005044289 A
- US 5484595 A
- WO 2007020382 A
- WO 2006122485 A
- WO 20061222454 A
- US 20030026854 A, Mr Zhao
- WO 2007005760 A
- KR 2001931
- CN 11040971
- WO 2005115421 A
- WO 2005105123 A
- WO 2004012496 A
- WO 2004012752 A
- WO 2004016106 A
- WO 2007057439 A
- WO 0121605 A
- WO 0152837 A
- WO 0206205 A
- WO 0121150 A
- WO 0151596 A
- FR 2822821
- FR 2857596
- WO 2005102259 A
- WO 9847479 A
- WO 2004112742 A
- WO 2008009709 A
- WO 2004050052 A
- WO 2004050079 A
- WO 2004112741 A
- WO 2008080974 A


### Littérature non-brevet citée dans la description


- **BROWN.** *Journal of photochemistry and photobiology B :biology,* 2001, vol. 63, 148-161